# EUROPEAN PATENT SPECIFICATION

(11) **EP 3 986 929 B1**
(45) Date of publication and mention of the grant of the patent: **20.11.2024**
(21) Application number: 20826775.7
(22) Date of filing: 17.06.2020
(51) Int. Cl.: C07K 16/06, G01N 33/53

(54) **ASSESSMENT OF DISEASE RISK FACTORS IN CORRELATION WITH NEU5GC INGESTED IN FOOD**
BEURTEILUNG VON KRANKHEITSRISIKOFAKTOREN IN ZUSAMMENHANG MIT ÜBER NAHRUNGSMITTEL AUFGENOMMENER NEU5GC
ÉVALUATION DE FACTEURS DE RISQUE DE MALADIE EN CORRÉLATION AVEC NEU5GC INGÉRÉ DANS DES ALIMENTS

(30) Priority: 18.06.2019 US 201962862728 P
(43) Date of publication of application: 27.04.2022
(73) Proprietor: Ramot at Tel-Aviv University Ltd., 6139201 Tel-Aviv (IL)
(72) Inventor: PADLER-KARAVANI, Vered, 6139201 Tel Aviv (IL); BASHIR, Salam, 6139201 Tel Aviv (IL); FEZEU, Leopold, 93430 Villetaneuse (FR)
(74) Representative: Straus, Alexander
(86) International application number: PCT/IL2020/050676
(87) International publication number: WO 2020/255134

(56) References cited:
- WO-A2-2005/010485
- US-A1- 2014 178 365
- SAMRAJ ANNIE N. ET AL: "A red meat-derived glycan promotes inflammation and cancer progression", PROCEEDINGS OF THE NATIONAL ACADEMY OF SCIENCES, vol. 112, no. 2, 13 January 2015 (2015-01-13), pages 542 - 547, XP055774077, ISSN: 0027-8424, DOI: 10.1073/pnas.1417508112
- LEVIATAN BEN-ARYE SHANI ET AL: "Differential Recognition of Diet-Derived Neu5Gc-Neoantigens on Glycan Microarrays by Carbohydrate-Specific Pooled Human IgG and IgA Antibodies", BIOCONJUGATE CHEMISTRY, vol. 30, no. 5, 17 April 2019 (2019-04-17), US, pages 1565 - 1574, XP093040427, ISSN: 1043-1802, Retrieved from the Internet <URL:https://pubs.acs.org/doi/pdf/10.1021/acs.bioconjchem.9b00273> DOI: 10.1021/acs.bioconjchem.9b00273
- BASHIR SALAM ET AL: "Presentation Mode of Glycans Affect Recognition of Human Serum anti-Neu5Gc IgG Antibodies", BIOCONJUGATE CHEMISTRY, vol. 30, no. 1, 30 November 2018 (2018-11-30), US, pages 161 - 168, XP093040432, ISSN: 1043-1802, Retrieved from the Internet <URL:https://pubs.acs.org/doi/pdf/10.1021/acs.bioconjchem.8b00817> DOI: 10.1021/acs.bioconjchem.8b00817
- SAMRAJ, ANNIE N. ET AL.: "A red meat-derived glycan promotes inflammation and cancer progression", PROCEEDINGS OF THE NATIONAL ACADEMY OF SCIENCES, vol. 112, no. 2, 13 January 2015 (2015-01-13), pages 542 - 547, XP055774077

## Description

### FIELD OF THE INVENTION

The present invention relates to methods for identifying a subject at increased risk of developing an *N*-glycolylneuraminic acid (Neu5Gc)-related disease or disorder, to methods of assessment of disease risk factors in correlation with consumption of Neu5Gc from food and antibodies against Neu5Gc.

### BACKGROUND OF THE INVENTION

Nutrition can dramatically affect health, and different dietary habits have been associated with various human diseases such as cancer, cardiovascular diseases, type II diabetes, obesity and hypertension. In particular, high consumption of red meat has frequently been suggested as a risk factor for human cancers and cardiovascular diseases. In some studies dairy also seems to be linked to such diseases, yet this association remains controversial. Although various mechanistic explanations have been proposed, none seem to be specific for red meat or dairy. Recently, the non-human carbohydrate *N*-glycolylneuraminic acid (Neu5Gc), that is present in mammalian-derived food (i.e. red meat and dairy), has been suggested as a possible risk factor in cancer, cardiovascular disease or chronic inflammation. Antibodies against Neu5Gc have been implicated as a new risk factor for colorectal cancer despite a lack of correlation to the consumption of Neu5Gc from food (Samraj et al., PLoS One. 2018;13: e0197464).

Neu5Gc is a common type of sialic acid in mammals. It is a nine-carbon negatively charged carbohydrate that can be synthesized by most mammals and is present as a terminal sugar moiety eventually found attached at the tips of carbohydrate chains (glycans), glycoproteins and glycolipids. Humans specifically cannot synthesize Neu5Gc due to a deletion in the *CMAH* gene that encodes the cytidine 5'-monophosphate-Neu5Ac hydroxylase. Yet, dietary-Neu5Gc that is consumed from the diet can be incorporated onto human cell surfaces, consequently displaying a broad assortment of immunogenic Neu5Gc-containing glycans. In fact, all humans examined thus far have a diverse collection of polyclonal anti-Neu5Gc antibodies. Thus, circulating anti-Neu5Gc antibodies continuously encounter Neu5Gc-containing epitopes on human tissues and have been proposed to lead to chronic inflammation termed "*xenosialitis*" that could potentially exacerbate various chronic-inflammation-mediated conditions as cancer and cardiovascular disease. Accordingly, xenosialitis induced in the human-like Neu5Gc-deficient *Cmah*^{*-*/*-*} mice exhibited a substantially higher incidence of carcinoma in the liver, with an elevated inflammatory response phenotype. Yet in mice, anti-Neu5Gc antibodies seem to have dual effects on cancer, in which a low dose promote tumor growth, while higher doses mediate tumor inhibition, effects that can switch even at only twofold concentrations of antibodies. Likewise, certain anti-Neu5Gc antibodies can actually serve as a carcinoma biomarker for human patients (Padler-Karavani et al., Cancer Res. 2011;71: 3352-3363), and have also been proposed to be used for cancer therapy. Altogether, the coexistence and interactions between Neu5Gc on human cells with circulating anti-Neu5Gc antibodies have been suggested to "tip the scales" in chronic-inflammation-meditated diseases through modulation of the inflammatory response characteristics.

The two most common sialic acids in mammals are *N*-acetylneuraminic acid (NeuSAc) and its hydroxylated form Neu5Gc, and their levels vary in different organisms and tissues. While Neu5Ac is a native 'self carbohydrate in humans, Neu5Gc is a non-human immunogenic carbohydrate. Neu5Gc seem to be abundant in red meat and dairy, while low in some fish, and non-existent in chicken. As discussed in Gao et al., (2017, PLOS ONE, 12(7) e0180768A), almost all people have circulating anti-Neu5Gc antibodies. According to Gao et al., the level of anti-Neu5Gc IgG as measured against a single synthetic target containing alpha-Neu5Gc-polyacrylamide is higher in men than women, however their level does not change with age or diet (see also Padler-Karavani et al., Glycobiology. 2008, 18(10):818-30). It was previously shown that an increase in anti-Neu5Gc antibodies levels that is not related to food, e.g. drug-induced increase, is not responsible to Neu5Gc related inflammations, at least by *in vitro* assay. Salama et al., (Transplantation, 2017;101:2501-2507) showed that none of the diverse diabetic patients treated with rabbit anti-thymocyte globulin had any adverse inflammation effects, despite the presence of high levels of drug-induced anti-Neu5Gc antibodies, even when these levels remained high for almost two years post treatment. A fairly simple method for assessment of the overall human anti-Neu5Gc antibodies was suggested by Padler-Karavani et al., (PLOS ONE, 2013, 8 93 e58443) against a collection of natural Neu5Gc-containing glycoproteins. Nevertheless, despite the extensive studies in several cohorts, up until now, the correlation between dietary uptake of Neu5Gc and the levels of anti-Neu5Gc antibodies could not be established (Samraj et al, 2014, Frontiers in Oncology Volume 4, Article 33 and Samraj PLoS One. 2018; 13(6): e0197464.)

There is an unmet need for simple and reliable diagnostic tools for identifying people having increased risk of developing a disease or condition related to consumption of Neu5Gc.

### SUMMARY OF THE INVENTION

According to the principles of this invention, for the first time it has been shown that there is a correlation between food consumption habits of a person and the level and diversity of anti-Neu5Gc antibodies. This correlation could be established only when meticulously performed data collection about a subject's food consumption habits was executed in combination with measurement of the total anti-Neu5Gc antibodies repertoire. This correlation and a score that may be calculated based on the antibody levels have a very important predictive value. It is now possible to link the amount of consumed Neu5Gc from the diet, to the amount of antibodies against Neu5Gc and their repertoire, and these associations may in turn be linked to an illness risk related to Neu5Gc-intake. This may be used in prognostics and prediction of treatment efficacy of these conditions. Moreover, based on these findings, it was possible to define differences in repertoires of anti-Neu5Gc antibodies between subjects who consumed high versus low amounts of Neu5Gc from food. This difference in the repertoire of antibodies may be used as a predictive tool for calculating the probability to develop a-disease or condition related to consumption of Neu5Gc from food and for identifying subjects having increased risk to develop such diseases. Additionally, the difference in the repertoire of antibodies may also be used to distinguish between people having an elevated levels of anti-Neu5Gc antibodies due to high consumption of Neu5Gc from food from those who have high level of anti-Neu5Gc antibodies as a result of exposure to factors that cause to increase of anti-Neu5Gc antibodies which are not related to food, such as drugs.

According to one aspect, the present invention provides a method for determining an antibody repertoire score in a biological sample obtained from a subject comprising: (i) evaluating the repertoire of the anti-Neu5Gc antibodies in the biological sample obtained from the subject; and (ii) calculating a ratio of antibodies against Neu5Gcα2-3-linked glycans to antibodies against Neu5Gcα2-6-linked glycans, wherein the calculated ratio is the antibody repertoire score and wherein the antibody repertoire score value above a control value is indicative to increased likelihood of developing a Neu5Gc related disease or disorder.

According to some embodiments, evaluating the repertoire of the anti-Neu5Gc antibodies in the biological sample comprises determining the amount of antibodies against a set of Neu5Gcα2-3-linked glycans and the the amount of antibodies against a set of Neu5Gcα2-6-linked glycans in said biological sample.

According to another aspect, the present invention provides a method for identifying a subject at increased risk of developing a Neu5Gc related disease or disorder comprising:
(i) calculating an antibody repertoire score; and (ii) comparing the obtained score to a control antibody repertoire score, wherein the value of said antibody repertoire score above the value of the control antibody repertoire score is predictive of an increased risk of developing the disease or disorder. Also provided is a method for identifying a subject at increased risk of developing a disease related to consumption of foods containing Neu5Gc, comprising: (i) calculating an antibody repertoire score; and (ii) calculating a ratio of antibodies against Neu5Gcα2-3-linked glycans to antibodies against Neu5Gcα2-6-linked glycans, wherein the calculated ratio is the antibody repertoire score and wherein the antibody repertoire score value above a control value is indicative to increased likelihood of developing a Neu5Gc related disease or disorder. According to some embodiments, the antibody repertoire score is calculated according to the method of the present invention. Thus, the present invention provides a method for identifying a subject at increased risk of developing a Neu5Gc related disease or disorder comprising: (i) evaluating the repertoire of the anti-Neu5Gc antibodies in the biological sample obtained from the subject; and (ii) calculating a ratio of antibodies against Neu5Gcα2-3-linked glycans to antibodies against Neu5Gcα2-6-linked glycans, and (iii) comparing the obtained score to a control antibody repertoire score, wherein the value of said antibody repertoire score above the value of the control antibody repertoire score is predictive to increased risk of developing the disease or disorder. According to any one of the above aspects and embodiments, the control antibody repertoire score is 0.9. According to some embodiments, the method further comprises informing the subject having an increased risk of developing the disease or disorder and providing dietary guidelines designed to reduce the risk.

According to any one of the aspects and embodiments of the present invention, the Neu5Gc related disease or disorder is selected from cancer, atherosclerotic cardiac disease, and chronic inflammatory conditions.

According to yet another aspect the present invention provides a diagnostic kit for evaluating the repertoire of the anti-Neu5Gc antibodies in a biological sample comprising:
a set of Neu5Gcα2-3-linked glycans;
a set of Neu5Gcα2-6-linked glycans; and
means for quantifying the amount of antibodies bound to Neu5Gcα2-3-linked glycans and to Neu5Gcα2-6-linked glycans.

According to some embodiments, the set of Neu5Gcα2-3-linked glycans comprises 3 or more of glycans 2, 8, 10, 12, 14, 16, 22, 26, 30, 34, 36, 40, 56, 58, 61, 63 as set forth in Table 1. According one embodiment, the set of Neu5Gcα2-3-linked glycans comprises at least 3 glycans of said list.

According to other embodiments, the set of Neu5Gcα2-6-linked glycans comprises two or more of glycans 4, 6, 18, 20, 24, 28, 32, 38 as set forth in Table 1. According one embodiment, the set of Neu5Gcα2-6-linked glycans comprises at least 3 glycans of said list.

According to some embodiments, the glycans are immobilized on a solid surface such as membrane or beads or dissolved in a solvent or buffer. According to some embodiments, the kit is configured to allow detection and quantification of said glycans. According to some embodiments, the glycans are marked by one detectable marker or by two or more different markers. According to some embodiments, the markers are configured to bind/adsorb to a surface.

According to any aspect and embodiment of the present invention, the determination of antibodies against Neu5Gc antibodies may be performed by any known methods. According to some embodiments, the method of detection comprises immunoassay or FACS. According to some embodiments, the immunoassay is ELISA.

According to some embodiments, the kit of the present invention is used to determine the antibody repertoire score. According to other embodiments, the kit is used in methods for identifying a subject at increased risk of developing a Neu5Gc related disease or disorder.

According to another aspect, the present invention provides a method for predicting a likelihood of developing a Neu5Gc related disease or disorder, comprising: (i) evaluating the repertoire of the anti-Neu5Gc antibodies in a biological sample obtained from a subject; (ii) calculating the ratio between antibodies against Neu5Gcα2-3-linked glycans and antibodies against Neu5Gcα2-6-linked glycans; and (iii) comparing the ratio obtained to the ratio in control subjects having low likelihood, wherein the change in the ratio compared to control subjects is predictive for developing the disease or disorder. According to some embodiments, the method further comprising measuring the level of total anti-Neu5Gc antibodies and incorporating the results in calculation of the likelihood. According to some embodiments, evaluating the repertoire of the anti-Neu5Gc antibodies comprises calculating an antibody repertoire score according to methods of the present invention.

### BRIEF DESCRIPTION OF THE FIGURES

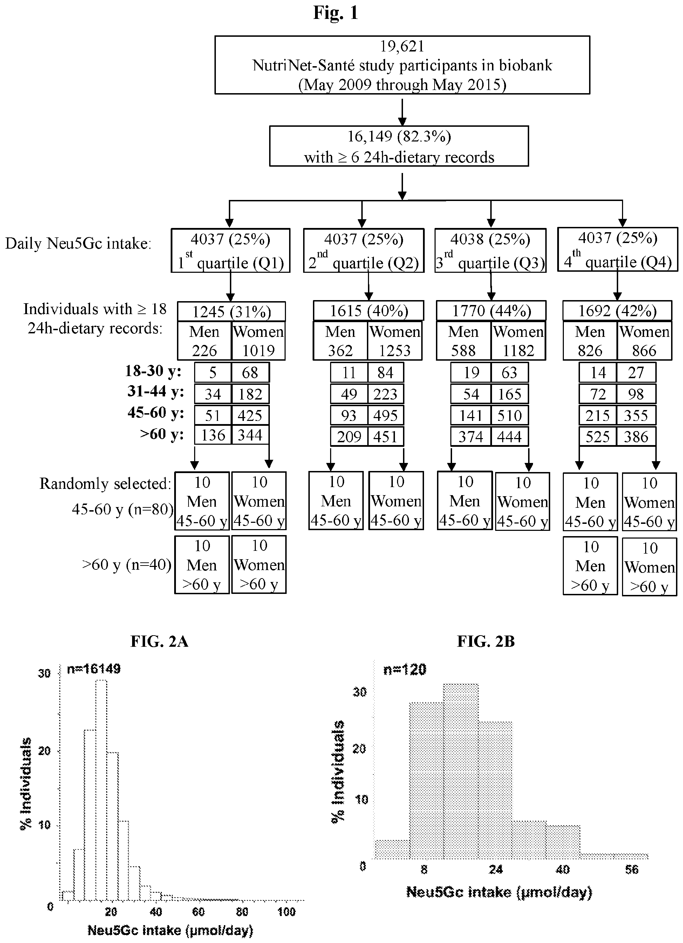

**Fig.** 1 shows the flow chart describing the selection of study cohort.

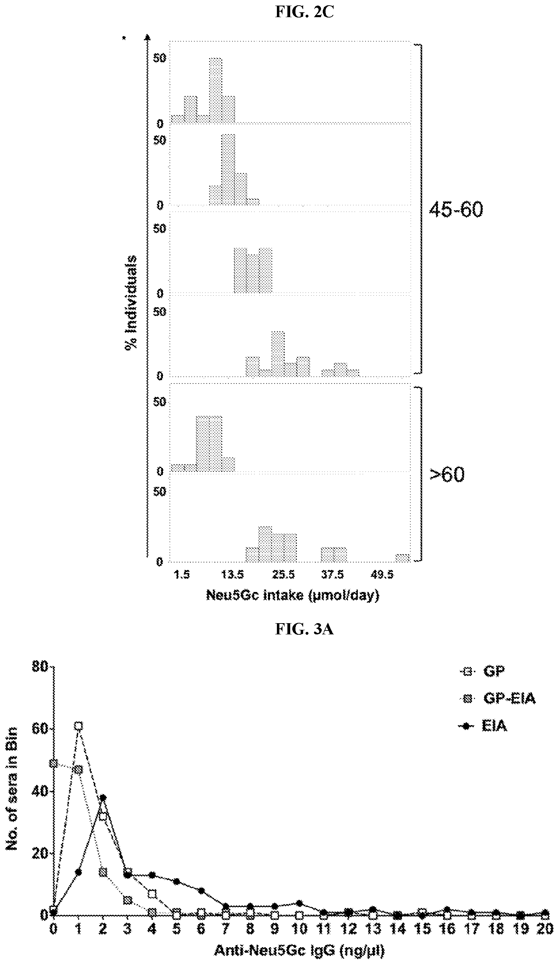

**Fig. 2** shows the daily Neu5Gc intake of subjects in NutriNet-Santé study cohort. **Fig. 2A** **-** distribution of NutriNet-Santé study participants (May 2009 through May 2015) according to daily Neu5Gc intake calculated from the total mean Neu5Gc of 24h-dietary records for each individual. **Fig. 2B** - diversity of daily Neu5Gc intake in the selected 120 individuals (out of 16149 examined). **Fig. 2C -** 10 men and 10 women were selected per Neu5Gc intake quartile by gender (age 45-60: Q1-Q4; age >60: Q1 and Q4), each with at least 18 dietary records.

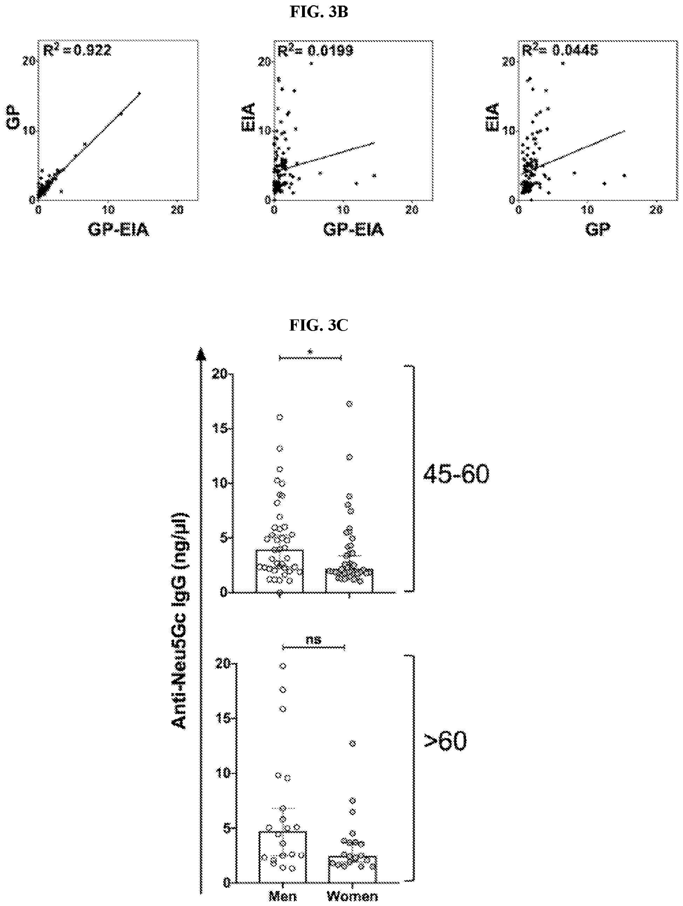

**Fig. 3** shows the measurements of anti-Neu5Gc IgG in 120 subjects of study cohort by ELISA. **Fig 3A** shows distribution of serum anti-Neu5Gc IgG levels quantified by three different ELISA methods against coated Neu5Gc-glycoproteins (EIA) or Neu5Gc-glycopeptides (GP) and GP-EIA. The IgG were then detected by HRP-anti-human IgG (H+L) (mean ± sem; GP: 2 ± 1.9 ng/µl, GP-EIA: 1.1 ± 1.9 ng/µl, EIA: 4.4 ± 3.9 ng/µl). **Fig. 3B** shows strong correlation between GP and GP-EIA assays, while no correlation between EIA and GP-EIA or GP assays (linear regression), **Fig. 3C** shows that the overall anti-Neu5Gc IgG (by EIA) were significantly higher in men compared to women aged 45-60, with a similar trend in the group aged >60 (median with 95% CI, Mann-Whitney test; *, p = 0.0397; ns, p=0.0822).

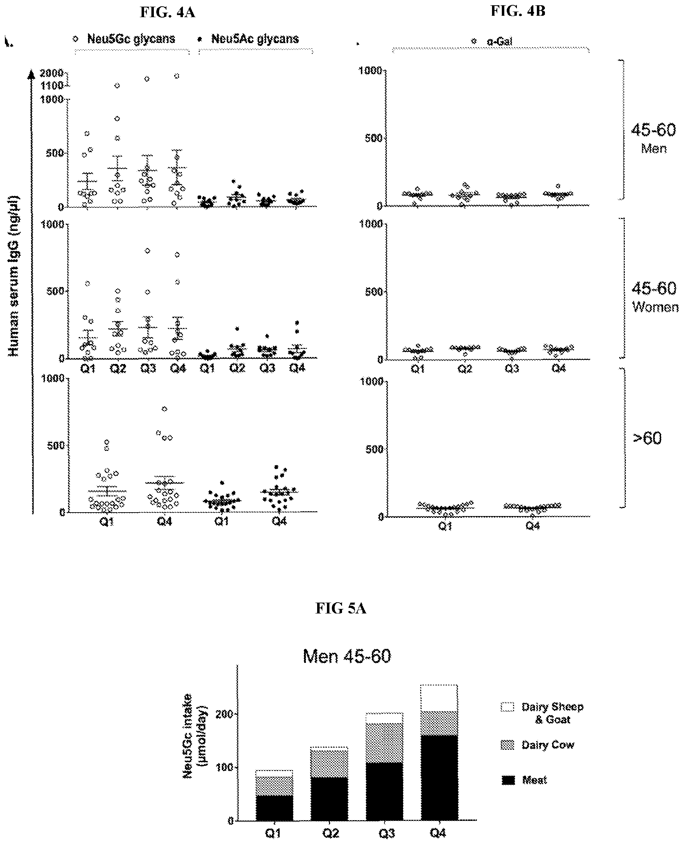

**Fig. 4** shows human IgG reactivity against Neu5Gc-glycans and Neu5Ac-glycans, and aGal by glycan microarrays. Human IgG reactivities in serum samples (n = 120) were assessed and values per serum sample were plotted according to quartiles of dietary Neu5Gc intake. **Fig. 4A** shows human serum IgG reactivities represented as the sum IgG response per individual against Neu5Gc-/Neu5Ac-glycans, per Neu5Gc intake quartile; it is shown a specific anti-Neu5Gc IgG reactivity (mean ± sem; Friedman ANOVA, p < 0.0001), with a trend for elevated anti-Neu5Gc IgG with higher dietary Neu5Gc levels. **Fig. 4B** shows human serum IgG reactivities per individual against aGal printed-glycan; no change in levels per Neu5Gc intake quartile was shown.

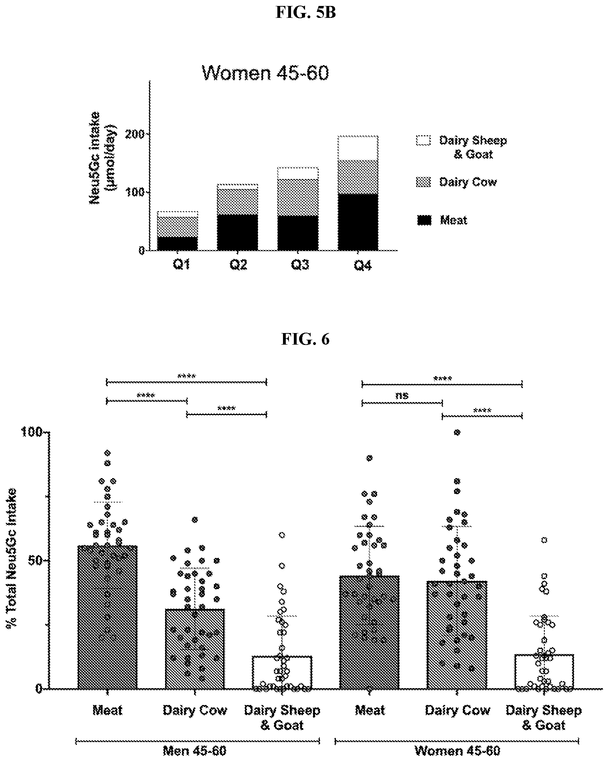

**Fig. 5** shows the shows stratification of men **(****Fig. 5A****)** and women **(****Fig. 5B****)** in age group 45-60 according to their total Neu5Gc-intake (Q1-Q4) and the contribution of dietary sources; a clear increase in Neu5Gc intake between quartiles, with dominance for red meat contribution was shown.
**Fig. 6** shows distribution of Neu5Gc intake by food source. In the 45-60 age group (40 men and 40 women), daily Neu5Gc intake was calculated per food source and plotted by gender. Each dot represents an individual donor subject (n=40 per column). Dietary Neu5Gc was dominantly contributed from red meat in men, while dairy in women.

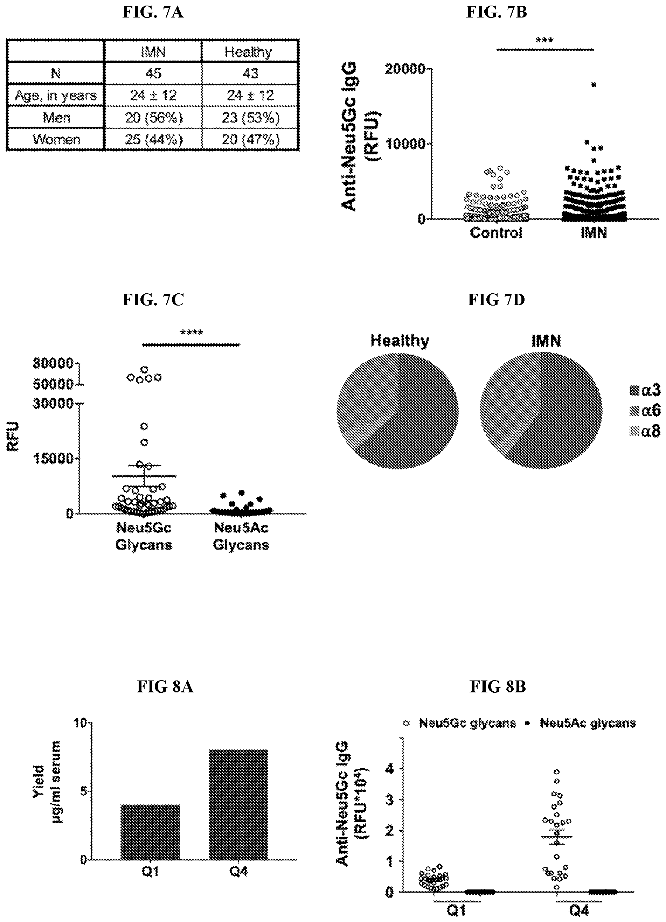

**Fig. 7** shows anti-Neu5Gc IgG response in patients with infectious mononucleosis and controls. **Fig. 7A** shows human serum samples obtained from patients with infectious mononucleosis (IMN) and age/gender matched healthy controls. **Fig. 7B** shows serum samples examined on sialoglycan microarrays printed with 24 Neu5Gc-glycans and their matching 24 Neu5Ac-glycans (at 1/100), then IgG reactivity detected with Cy3-anti-human IgG in relative fluorescent units (RFU), showing higher anti-Neu5Gc IgG reactivity in IMN patients compared to controls (each dot is IgG response per Neu5Gc-glycan per serum; mean ± sem; unpaired Mann-Whitney test; *** p = 0.0003). **Fig. 7C** shows IgG reactivity of extremely high specificity against Neu5Gc-glycans, with minimal reactivity against NeuSAc-glycans (each dot represents the sum IgG response against all glycans per serum; Wilcoxon matched paired test, **** p < 0.0001). Fig. 7D shows Pie charts of the sum anti-Neu5Gc IgG response, divided according to reactivity against Neu5Gc-glycans with different Sia-linkages (α3, α6, α8). No difference between the two groups was seen, supporting no altered diversity.

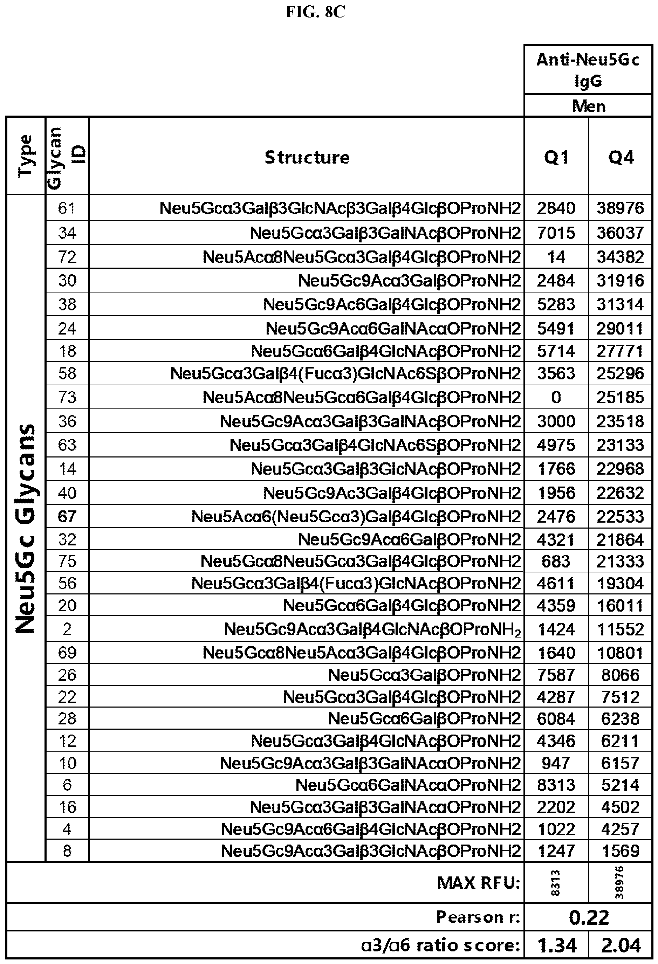

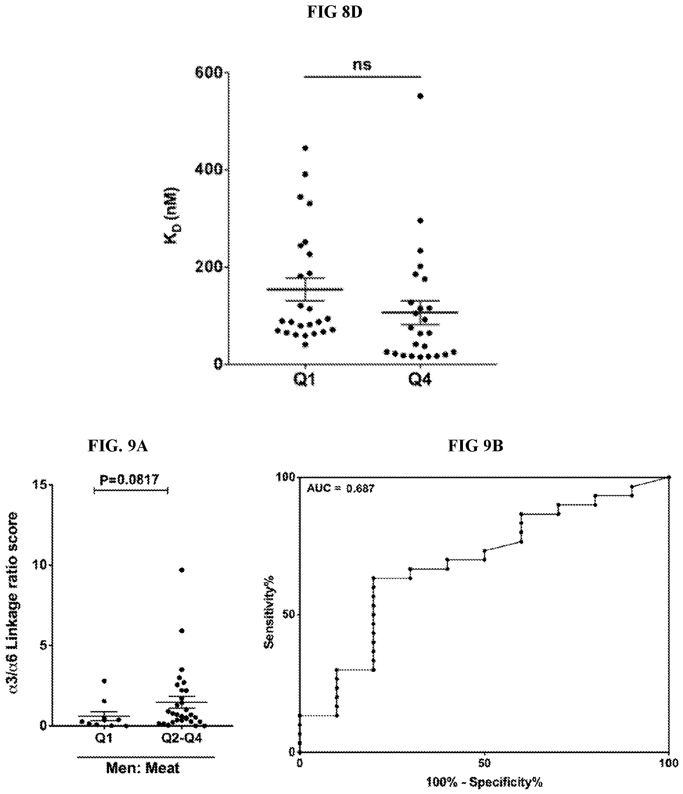

**Fig. 8** shows characteristics of affinity-purified anti-Neu5Gc antibodies of men 45-60. Anti-Neu5Gc antibodies were affinity-purified from pooled sera of men aged 45-60 consuming low (Q1) or high (Q4) levels of Neu5Gc from red meat (5.39 ml of Q1, and 6.8 ml of Q4; n=10 per group). **Fig 8A** **-** total antibody yield was higher in Q4 compared to Q1 (8.01 µg/ml serum versus 4.01 µg/ml serum, respectively). **Figs. 8B-8C** show IgG reactivity examined on sialoglycan microarrays (2 µg/block; detected with Cy3-anti-human IgG). It revealed extremely high specificity against Neu5Gc-glycans, and no reactivity against their counterpart Neu5Ac-glycans (**Fig. 8B****;** each dot is IgG response per glycan), with a clear change in diversity of glycan recognition in Q4 compared to Q1 (**Fig. 8C****;** Pearson r=0.22). Fig. 8D shows affinity (K_{D}) per glycan calculated from anti-Neu5Gc IgGs reactivity examined on sialoglycan microarrays at 16 serial dilutions (40 - 4.9 × 10⁻³ ng/µl; 266.7 - 0.033 nM; non-linear fit with one-site specific binding). It showed no change in affinities for higher Neu5Gc intake (mean ± sem; t-test).
**Fig. 9** shows a ratio of anti-Neu5Gc IgG reactivity against glycans with α3-linkage versus a6-linkage in men 45-60. **Fig. 9A** **-** The α3/α6 linkage ratio score in Q1 (n=10) appeared to be lower than that calculated for Q2-Q4 (n=30), (Mann-Whitney test; P=0.0817; Q1; 0.6080 ± 0.2806, Q4; 1.472 ± 0.3722; mean ± sem). Similar results were obtained in comparison between Q1 to Q4 (P=0.0892; Q1: 0.6080 ± 0.2806, Q4: 1.384 ± 0.3961). **Fig. 9B** - shows ROC curve analysis of α3/α6 linkage ratio score in Q1 (n=10) compared to Q2-Q4 (n=30).

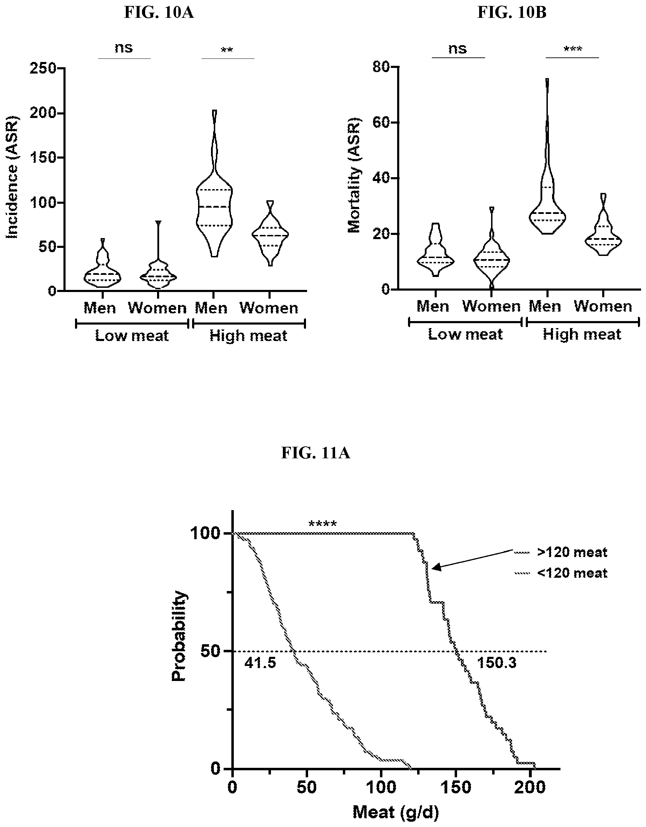

**Fig. 10** shows incidence of colorectal cancer **(****Fig. 10A****)** and mortality **(****Fig. 10B****)** per nation of highest and lowest quartiles of meat consumption (n=38 each) divided by gender.

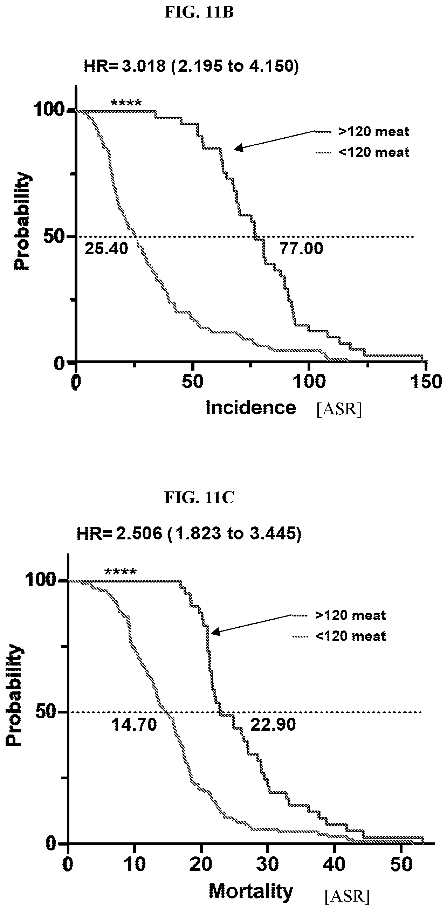

**Fig. 11** Shows the distribution (probability) of meat consumption, or incidence and mortality rates due to colorectal cancer (CRC at age-standardized rates; ASR per 100,000 person-years, including colon, rectum, anus cancers) in different countries comparing two groups by mean intake of meat of above/below 120 gr meat/day. The data is from 152 countries total, 41 countries consumed meat over 120 g/day while 111 countries consumed below 120 g/day. Fig 11A-probability (percent of countries in that group) of countries below/above 120 gr/day meat consumption, **Fig. 11B** - probability of incidence of CRC and **Fig. 11C** - probability of mortality due to CRC [Survival proportions calculated by Log-rank (Mantel-Cox) test; **** p=0.0001; Median survival marked in dotted lines; Hazard Ratio (HR) and their 95% CI of ratio were calculated by logrank method].

### DETAILED DESCRIPTION OF THE INVENTION

Provided are diagnostic tools and kits for identifying subjects having an increased risk to develop a disease or disorder related to consumption of Neu5Gc from food and methods for predicting the likelihood for developing such a disease or disorder. According to one aspect, the present invention provides a method for determining an antibody repertoire score in a biological sample comprising:
(i) evaluating the repertoire of anti-Neu5Gc antibodies in the biological sample; and
(ii) calculating a ratio of antibodies against Neu5Gcα2-3-linked glycans to antibodies against Neu5Gcα2-6-linked glycans,
wherein the calculated ratio is the antibody repertoire score.

As used herein, the term "biological sample" refers to any biological fluid or tissue obtained from a subject in which antibodies may be detected and quantified. According to some embodiments, the biological sample is blood. According to one embodiments, the biological sample is serum. According to another embodiments, the biological sample is serum.

According to some embodiments, the antibody repertoire score value above a control value is indicative for increased likelihood of developing a Neu5Gc related disease or disorder.

The term "antibody repertoire score" as used herein refers to a ratio of antibodies against Neu5Gcα2-3-linked glycans to antibodies against Neu5Gcα2-6-linked glycans in the biological sample of a subject in which said antibodies can be detected. According to some embodiments, the antibodies are not-treated, i.e. non-purified antibodies. The term "antibodies against target" has the meaning of antibody that binds specifically to said target/antigen.

Sialic acids (Sia) have common three functional groups: a carboxylate (at carbon position C-1) attached to the C-2 anomeric carbon (α-keto; carries a carbonyl in the Sia open chain form), a 3-carbon side chain (C-7, C-8, C-9) projecting out of the 6-carbon ring of a cyclic Sia, and an acylated amino group. The immense structural diversity of Sia containing glycans stems from the variety of terminal Sia (e.g. Neu5Gc, NeuSAc), Sia-linkages, underlying glycans (sialoglycans), glycan-carrier (sialoglucoconugates; e.g. protein or lipid) and their spatial organization (saccharide patches by proximity of different glycans). In terms of linkage diversity, Sia is alpha-linked through its C-2 position to galactose (at C-3/4/6; Siaα2-3/4/6Gal), *N* acetylgalactosamine (at C-6; Siaα2-6GalNac), N-acetylglucosamine (at C-4/6; Siaα2-4/6GlcNAc), or to another Sia (at C-8/9; Siaα2-8/9Sia). Likewise, structurally diverse Neu5Gc-glycans can be generated based on their linkages to underlying glycans. Neu5Gc alpha-linked through its C-2 position to the C-3/6/8 positions of an underlying carbohydrate are referred as Neu5Gcα2-3-linked, Neu5Gcα2-6-linked Neu5Gcα2-8-linked glycans, respectively.

The term "binds specifically" refers to an interaction wherein the antibody associates with a specific glycan more frequently or rapidly, or with greater duration or affinity, or any combination of the above, than with other structurally related/similar glycans, and preferably do not cross-react with other glycans.

The term "Neu5Gc" and "*N*-glycolylneuraminic acid" are used herein interchangeably and refer to a sialic acid molecule having the structure as presented in Formula I and has CAS number 1113-83-3.

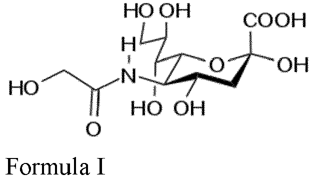

Neu5Gc is found in most non-human mammals but cannot be synthesized in human. The term refers also to any derivatives of Neu5Gc.

The term "total anti-Neu5Gc antibodies" refers to antibodies against a plurality of different Neu5Gc-glycans, preferably against a plurality of the Neu5Gc-glycans present in mammalian serum, cells or tissues. According to some embodiments, the term refers to the antibodies reactive against mammalian serum Neu5Gc-glycoproteins. According to one embodiment, the mammal is selected from primates, livestock animals (including bovines, porcine, etc.), companion animals (e.g., canines, felines, etc.) and rodents (e.g., mice and rats). According to one embodiment, the term refers to the antibodies reactive against mice serum Neu5Gc-glycoproteins. According to other embodiments, the term refers to antibodies reactive against a plurality of serum glycopeptides, such as mouse serum glycopeptides. According to other embodiments, the term refers to antibodies reactive against a plurality of serum Neu5Gc-glycans present in human subject.

According to some embodiments, the biological sample is any sample in which antibodies against Neu5Gc may be detected. According to some embodiments, the biological sample is selected from serum and plasma. Thus, according to some embodiments, the amount of the total anti-Neu5Gc antibodies is measured in a serum or from plasma of a subject.

According to some embodiments, the subject is a human subject. According to one embodiment, the subject is a man. According to other embodiments, the subjects is a woman. According some embodiments, the subject is up to 60 years old. According to one embodiment, the subject is up to 65, up to 67 or up to 70 years old. According to some embodiments, the subject is 30-60 or 40-60, 40-65, 40-70, 60-65 or 60-70 years old. According to some embodiments, the subject is 18-30, or under 18 old.

According to one embodiment, evaluating the repertoire of the anti-Neu5Gc antibodies comprises differentiating the antibodies according to the type of Neu5Gc-glycan epitope they bind. According to some embodiments, evaluating the repertoire of the anti-Neu5Gc antibodies comprises assessing the amount, i.e. determining the amount, of antibodies against Neu5Gcα2-3-linked glycans and the amount of antibodies against Neu5Gcα2-6-linked glycans in the biological sample. According to some embodiments, the Neu5Gcα2-3-linked glycans and Neu5Gcα2-6-linked glycans refer to all Neu5Gcα2-3-linked glycans and Neu5Gcα2-6-linked glycans to which antibodies may be produced and measured. According to some embodiments, the amount of antibodies against Neu5Gcα2-8-linked glycans is determined as well. It is understood that a repertoire of antibodies against Neu5Gc glycans correlates to repertoire of recognized Neu5Gc antigens.

According to some embodiments, the glycans are as defined in **Table 1.** The term "collection of Neu5Gcα2-3-linked glycans" refers to all Neu5Gcα2-3-linked glycans presented in Table 1, i.e. to glycans having glycan ID numbers 2, 8, 10, 12, 14, 16, 22, 26, 30, 34, 36, 40, 56, 58, 61, 63 as in Table 1 (total 16 glycans). The term "collection of Neu5Gcα2-6-linked glycans" refers to all Neu5Gcα2-6-linked glycans presented in Table 1, i.e. to glycans having glycan ID numbers 4, 6, 18, 20, 24, 28, 32, 38 in Table 1 (total 8 glycans). According to some embodiments, evaluating the repertoire of the anti-Neu5Gc antibodies comprises evaluating the amount of antibodies against a plurality of Neu5Gcα2-3-linked glycans, which form is a subset (set) of the collection of Neu5Gcα2-3-linked glycans and evaluating the amount of antibodies against a plurality of Neu5Gcα2-3-linked glycans which form as subset (set) of the collection of Neu5Gcα2-3-linked glycans. The term "set of Neu5Gcα2-6-linked glycans" as used herein refers to a subset of the collection of Neu5Gcα2-6-linked glycans. The term "set of Neu5Gcα2-3-linked glycans" as used herein refers to a subset of the collection of Neu5Gcα2-3-linked glycans. According to some embodiments, the set of glycan is selected in such a way that determining the amount of antibodies against glycans of said set provides a diagnostic value. According to some embodiments, a set of Neu5Gcα2-3-linked glycans comprises 3, 4, 5, 6, 7, 8, 9, 10, 11, 12, 13, 14 or 15 glycans of the collection of Neu5Gcα2-3-linked glycans as defined hereinabove. According to some embodiments, a set of Neu5Gcα2-6-linked glycans comprises 3, 4, 5, 6, or 7 glycans of the collection of Neu5Gcα2-6-linked glycans as defined hereinabove.

According to one embodiment, the set of Neu5Gcα2-3-linked glycans comprises at least at least 3, at least 4, at least 5, at least 6, at least 7, at least 8, at least 9 or at least 10 glycans of the set of Neu5Gcα2-3-linked glycans. According to one embodiment, the set of Neu5Gcα2-6-linked glycans comprises at least at least 3, at least 4, at least 5, at least 6, or at least 7 glycans of the set of Neu5Gcα2-6-linked glycans. According to some embodiments, the set of Neu5Gcα2-3-linked glycans comprises at least 3 glycans of the set of Neu5Gcα2-3-linked glycans and the set of Neu5Gcα2-6-linked glycans comprises at least 3 glycans of the set of Neu5Gcα2-6-linked glycans.

According to one embodiment, the set of Neu5Gcα2-3-linked glycans comprises at least 4, 5, 6, 7 or 8 glycans of the set of Neu5Gcα2-3-linked glycans and the set of Neu5Gcα2-6-linked glycans comprises at least 3, 4, 5 or 6 glycans of the set of Neu5Gcα2-6-linked glycans. According to some embodiments, the set of Neu5Gcα2-3-linked glycans comprises at least 5 glycans of the set of Neu5Gcα2-3-linked glycans and the minimal set of Neu5Gcα2-6-linked glycans comprises at least 5 glycans of the set of Neu5Gcα2-6-linked glycans.

According to some embodiments, evaluating the repertoire of the anti-Neu5Gc antibodies in the biological sample obtained from the subject comprises determining the amount of antibodies against a set of Neu5Gcα2-3-linked glycans and against a set of Neu5Gcα2-6-linked glycans.

According to some embodiments, evaluating the repertoire of the anti-Neu5Gc antibodies in the biological sample obtained from the subject comprises determining the amount of antibodies against all glycans of the collection of Neu5Gcα2-3-linked glycans and against all glycans of the collection of Neu5Gcα2-6-linked glycans. In such a case the set of Neu5Gcα2-3-linked glycans and the set of Neu5Gcα2-6-linked glycans are also the collection of Neu5Gcα2-3-linked glycans and of Neu5Gcα2-6-linked glycans, respectively.

Any known method for determining the amount of antibodies against the sets of Neu5Gcα2-3-linked and Neu5Gcα2-6-linked glycans may be used. Measuring the amount of antibodies may be performed by any known method. According to some embodiments, measuring the level of anti-Neu5Gc antibodies is effected by an immunoassay methods. According to some embodiments, the immunoassay is ELISA. According to other embodiments, the immunoassay method is selected from ELISA inhibition assay against a collection or a set of Neu5Gc-glycoproteins, ELISA against a collection or a set of Neu5Gc-glycopeptides and a sialoglycan microarray with a collection or a set of Neu5Gc-glycans. According to other embodiments, the detection of the amount of antibodies may be performed by FACS. According to another embodiment, the method is lateral flow.

According to some embodiments, glycans may be immobilized on a solid surface. According to some embodiments, the solid phase is a membrane. According to another embodiment, the solid phase is a polymers. Non limiting examples of solid phases are nitrocellulose, polyvinylidene fluoride (PVDF); hydrophobic (Charge-modified) nylon and polyethersulfone (PESU). Alternatively, the solid phase may be a woven meshes, synthetic nonwovens, cellulose and glass fiber. According to alternative embodiments, the glycans may be dissolved in a solvent.

According to some embodiments, the glycans of the set of Neu5Gcα2-3-linked glycans and the glycans of the set of Neu5Gcα2-6-linked glycans are marked by one marker. According to other embodiments, the glycans of the two sets are marked by more than one marker. According to one embodiment, the glycans of the set of Neu5Gcα2-3-linked glycans and the glycans of the set of Neu5Gcα2-6-linked glycans are marked by different markers. According to some embodiments, the marker(s) allow quantifying separately antibodies bound to each set. According to some embodiments, the marker(s) allow quantifying separately antibodies specifically binding or capable of specifically binding to glycans of each set. According to such embodiments, the markers are configured to bind to a surface. According to some arrangements, the glycans of two sets are located in one compartment. According to some embodiments, marking of the glycans is aimed to allow quantifying Neu5Gcα2-6-linked glycans and Neu5Gcα2-3-linked glycans.

According to other embodiments, the glycans of two sets are located in different compartments. In such arrangements, only one marker may be used.

Any marker known in art may be used. According to some embodiments, the marker is a florescent marker. According to some embodiments, the marker is selected from avidin, biotin, and streptavidin.

According to some embodiments, determining an antibody repertoire score comprises calculation or assessment of a ratio between antibodies against Neu5Gcα2-3-linked glycans and antibodies against Neu5Gcα2-6-linked glycans (anti-Neu5Gc α3:α6 ratio).

According to another aspect, the present invention provides a method for identifying a subject at increased risk of developing a Neu5Gc related disease or disorder comprising:
(i) evaluating the repertoire of the anti-Neu5Gc antibodies in a biological sample obtained from the subject;
(ii) calculating a ratio of antibodies against Neu5Gcα2-3-linked glycans to antibodies against Neu5Gcα2-6-linked glycans;
(iii) comparing the obtained ratio to a control ratio,
wherein the value of said ratio above the value of the control ratio is predictive to increased risk of developing the disease or disorder.

According to some embodiments, present invention provides a method for identifying a subject at increased risk of developing a Neu5Gc related disease or disorder, the method comprises determining an antibody repertoire score in a biological sample and comparing the obtained antibody repertoire score to a control antibody repertoire score, wherein the value of said antibody repertoire score above the control value is predictive to increased risk of developing the disease or disorder. According to some embodiments, determining the antibody repertoire score is performed as described in any one of the above aspects and embodiments. According to some embodiments, the biological samples is obtained from a subject.

The term "control antibody repertoire score" as used herein refers to a mean antibody repertoire score obtained in subjects consuming food with low content of Neu5Gc, i.e. in subjects consuming no more than 9500 nmol/day of Neu5Gc.

According to any one of the above embodiments and aspects, the control ratio or control antibody repertoire score is 0.6. According to some embodiments, the control ratio or score is 0.7. According to other embodiments, the control ratio or score is 0.8. According to anther embodiment, the control score is 0.9. According to yet another embodiment, the control ratio or score is 1.0. According to certain embodiments, the control ratio or score is 1.2.

According some embodiments, the control ratio or control antibody repertoire score is 0.6 for male subjects. According to other embodiments, the control ratio or control antibody repertoire score is 0.7 for male subjects. According to certain embodiments, the control ratio or control antibody repertoire score is 0.8 for male subjects.

According other embodiments, the control ratio or control antibody repertoire score is 0.8 for female subjects. According to some above embodiments, the control ratio or control antibody repertoire score is 0.9 for female subjects. According to one embodiment, the control ratio or control antibody repertoire score is 1.0 for female subjects.

According to some embodiments, the present invention provides a method for identifying a subject at increased risk of developing a Neu5Gc related disease or disorder comprising calculating the antibody repertoire score as described in any one of the above aspects and embodiments, wherein the antibody repertoire score above 0.9 is predictive to increased risk of developing the disease or disorder.

According to some embodiments, the method further comprising measuring the level of total anti-Neu5Gc antibodies and incorporating the results in calculation of the likelihood of developing the disease or disorder.

According to some embodiments, the method further comprising informing the subject having an increased risk of developing the disease or disorder and providing dietary guidelines designed to reduce the risk. According to some embodiments, the dietary guidelines comprises guidance to consume no more than 10 µmol/day of Neu5Gc. According to other embodiments, the dietary guidelines comprises guidance to consume no more than 9.5 µmol/day of Neu5Gc. According to some embodiments, the dietary guidelines comprises guidance to consume no more than 9.0, no more that 8.5 or no more than µmol/day of Neu5Gc. According to some embodiments, the dietary guidelines comprises guidance to consume no more than 150 gram (gm) of meat per day. According to other embodiments, the dietary guidelines comprises guidance to consume no more than 130 gm of meat per day. According to certain embodiments, the dietary guidelines comprises guidance to consume no more than 120 gm of meat per day. According to another embodiment, the dietary guidelines comprises guidance to consume no more than 100 gm of meat per day. According to yet another embodiment, the dietary guidelines comprises guidance to consume no more than 90 gm of meat per day. According to some embodiments, the meat is a beef. According to some embodiments, the meat is other than beef and the higher recommended amount is calculated using **Table 6.**

According to some embodiments, the dietary guidelines are based on Neu5Gc content in food as disclosed in **Table 6.**

According to any one of the above aspects and embodiments, measuring the level of anti-Neu5Gc antibodies is performed by any known methods, e.g. by immunoassay. According to some embodiments, the immunoassay is ELISA. According to some embodiments, the immunoassay is selected from ELISA inhibition assay against a collection or a set of Neu5Gc-glycoproteins, ELISA against a collection or a set of Neu5Gc-glycopeptides and ELISA against a collection or a set of Neu5Gc-glycans e.g. in microarray.

According to any one of the above aspects and embodiments, the biological sample is blood or serum. According to some embodiments, the biological sample is obtainable from a subject. According to some embodiments, the method according to any one of the above aspects and embodiments, comprises obtaining the biological sample from the subject.

According to some embodiments, the present invention provides a method of preventing development of a Neu5Gc related disease or disorder in a subject in need thereof comprising consulting to said subject consuming no more than 10 µmol/day of Neu5Gc. According to some embodiments, the method comprises consulting to consume no more than 10 µmol/day of Neu5Gc. According to other embodiments, the method comprises consulting to consume no more than 9.5 µmol/day of Neu5Gc. According to some embodiments the method comprises consulting to consume no more than 9.0, no more that 8.5 or no more than µmol/day of Neu5Gc. According to some embodiments, the method comprises consulting to consume no more than 150 gm of meat per day. According to other embodiments, the method comprises consulting to consume no more than 130 gm of meat per day. According to certain embodiments, the method comprises consulting to consume no more than 120 gm of meat per day. According to another embodiment, the method comprises consulting to consume no more than 100 gm of meat per day. According to yet another embodiment, the method comprises consulting to consume no more than 90 gm of meat per day. According to some embodiments, the meat is a beef meat. According to some embodiments, the meat is other than beef meat and the higher recommended amount is calculated using Table 6. According to some embodiments, the wherein the subject is a subject having an increases risk to develop the Neu5Gc related disease or disorder as determined by the methods according to any one of the above aspects and embodiments.

As used herein, the term "preventing" when used in relation to a condition, refers to an action such as diagnosis that reduces the frequency of, or delays the onset of, symptoms of a medical condition in a subject relative to a subject that did not undergo the action. As used herein, the term "preventing" or "prevention" includes the prevention of the recurrence, spread or onset of a disease in a patient, such as cancer prevention of cancer onset.

According to some embodiments, the terms "acquiring" and "developing" referring to a disease, disorder or condition may be used interchangeably.

According to some embodiments, the present invention provides dietary guidelines for use in preventing development of a Neu5Gc related disease or disorder in a subject in need thereof said dietary guidelines comprise consulting to said subject consuming no more than 10 µmol/day of Neu5Gc. According to some embodiments, the subject is a subject having an increases risk to develop the Neu5Gc related disease or disorder as determined by the methods according to any one of the above aspects and embodiments.

According to some embodiments, the dietary guidelines are based on Neu5Gc content in food as disclosed in Table 6.

According to any one of the above aspects and embodiments, the Neu5Gc related disease or disorder is selected from atherosclerotic cardiac disease, cancer and chronic inflammatory conditions. According to some embodiments, the Neu5Gc related disease is cancer. According to some embodiments, cancer is carcinoma. According to one embodiment, the cancer is selected from gastric, colorectal, pancreatic, prostate, oral, esophageal, small intestinal, hepatic, ovarian, endometrial, and breast cancer. According to one embodiment, the cancer is selected from colorectal cancer and breast cancer. According to one embodiment, the cancer is colorectal cancer. According to another embodiment, the cancer is breast cancer.

Thus, according to some embodiments, the present invention provides a method for preventing cancer in a subject in need thereof comprising consulting to the subject consuming no more than 10 µmol/day of Neu5Gc. According to some embodiments, the present invention provides a method for preventing colorectal cancer in a subject in need thereof comprising consulting to the subject consuming no more than 10 µmol/day of Neu5Gc. According to other embodiments, the present invention provides a method for preventing breast cancer in a subject in need thereof comprising consulting to the subject consuming no more than 10 µmol/day of Neu5Gc. According to other embodiments, the present invention provides a method for preventing lung cancer in a subject in need thereof comprising consulting to the subject consuming no more than 10 µmol/day of Neu5Gc.

According to another aspect, the present invention provides a diagnostic kit for evaluating the repertoire of the anti-Neu5Gc antibodies in a biological sample comprising:
a set of Neu5Gcα2-3-linked glycans;
a set of Neu5Gcα2-6-linked glycans; and
means for quantifying the amount of antibodies bound to Neu5Gcα2-3-linked glycans and to Neu5Gcα2-6-linked glycans.

According to one embodiment, the set of Neu5Gcα2-3-linked glycans comprises at least 3 glycans of the the collection of Neu5Gcα2-3-linked glycans as defined herein above. According to one embodiment, the set of of Neu5Gcα2-3-linked glycans comprises at least 3 glycans selected from glycans having ID number 2, 8, 10, 12, 14, 16, 22, 26, 30, 34, 36, 40, 56, 58, 61, 63 as set forth in Table 1. According to another embodiment, the set of Neu5Gc α2-6-linked glycans comprises at least 3 of the glycans of the collection of Neu5Gcα2-6-linked glycans as defined herein above. According to some embodiments, the set of Neu5Gcα2-6-linked glycans comprises at least 3 of the glycans selected from glycans having ID number 4, 6, 18, 20, 24, 28, 32, and 38 as set forth in Table 1. According to one embodiment, the set of Neu5Gcα2-3-linked glycans comprises at least 4, 5, 6, 7, 8, 9, 10, 11 or 12 glycans of the collection of Neu5Gcα2-3-linked glycans and the set of Neu5Gcα2-6-linked glycans comprises at least 3, 4, 5 or 6 glycans of the collection of Neu5Gcα2-6-linked glycans.

According to one embodiment, the set of Neu5Gcα2-3-linked glycans comprises at least 5 glycans of as set of glycans having ID number 2, 8, 10, 12, 14, 16, 22, 26, 30, 34, 36, 40, 56, 58, 61, 63 as set forth in Table 1 and the set of Neu5Gcα2-6-linked glycans comprises at least 5 of the glycans of a set of glycans having ID number 4, 6, 18, 20, 24, 28, 32, and 38 as set forth in Table 1.

According to some embodiments, wherein the glycans are immobilized on a solid surface. Any solid surface may be used such as chip or microarray. According to some embodiments, the solid phase is a membrane. According to another embodiment, the solid phase is a polymers. Non limiting examples of solid phases are nitrocellulose, polyvinylidene fluoride (PVDF); hydrophobic (Charge-modified) nylon and polyethersulfone (PESU). Alternatively, the solid phase may be a woven meshes, synthetic nonwovens, cellulose and glass fiber.

According to an alternative embodiments, the glycans are dissolved in a solvent. According to some embodiments, the glycans are linked to beads.

According to some embodiments, the glycans of the set of Neu5Gcα2-3-linked glycans and the glycans of the set of Neu5Gcα2-6-linked glycans are marked by one or more markers. According to one embodiment, the glycans of the set of Neu5Gcα2-3-linked glycans and the glycans of the set of Neu5Gcα2-6-linked glycans are marked by different markers. According to some embodiments, the marker(s) allow quantifying separately antibodies bound to each set. According to one embodiment, the markers are configured to bind to a surface.

Any marker known in art may be used. According to some embodiments, the markers are florescent markers. According to some embodiments, the marker is selected from avidin, biotin, and streptavidin.

According to some embodiments, the glycans of the set of Neu5Gcα2-3-linked glycans and the glycans of the set of Neu5Gcα2-6-linked glycans are bound to beads such as magnetic bead. According to some embodiments, the beads for the set of Neu5Gcα2-3-linked glycans and beads of the set of Neu5Gcα2-6-linked glycans may be separated. According to some embodiments, the two sets of Neu5Gc glycans, i.e. set of Neu5Gcα2-3-linked glycans and the set of Neu5Gcα2-6-linked glycans, are placed in one compartment. According to another embodiment, the two sets are placed in two different compartments. According to some embodiments, when the two sets are placed in two compartments, only one marker can be used.

According some embodiments, the kit comprises means for quantifying the amount of antibodies bound to Neu5Gcα2-3-linked glycans and to Neu5Gcα2-6-linked glycans. According to one embodiment, the means is an immunoassay. According to some embodiments, the immunoassay is ELISA. According to other embodiments, the means is FACS. According to some embodiments, the means is lateral flow.

According to some embodiments, the kit comprises means for determining the ratio between antibodies bound to Neu5Gcα2-3-linked glycans and to Neu5Gcα2-6-linked glycans, i.e. the antibody repertoire score. According to some embodiments, the means allow quantifying the signals obtained from the two sets upon binding of antibodies and comparing the signals.

According to some embodiments, the kit is useful in the methods for determining the antibody repertoire score in a biological sample obtained from a subject, according to any one of the above aspects and embodiments. According to another embodiment, the kit is useful in a methods for identifying a subject at increased risk of developing a Neu5Gc related disease or disorder as described in any one of the above aspects and embodiments. Thus, the kit is a diagnostic kit.

According to another aspect, the present invention provides a method for predicting an increased likelihood of developing a Neu5Gc related disease or disorder, the method comprises calculating an antibody repertoire score according to the method described in any one of the above aspects and embodiments, and further calculating the intake of NeuSGs based on a collected data on food consumption habits of the subject and combining the results with the antibody repertoire score to calculate an joined likelihood predictive of developing the disease.

As used herein, the term "food" includes any nourishing substance that is eaten or drunk. Foods include solids, liquids, gels, and slurries.

According to some embodiments, the amount of anti-Neu5Gc antibodies may be measured by ELISA, using a microarray or any other known method.

According to some embodiments, collecting data on actual food consumption of a subject comprises at least three 24-hour dietary records of the consumed food, wherein at least 3 of said records are collected at non-consecutive days.

According to some embodiments, the collecting data comprises recording of at least 4, at least 5, at least 6, at least 7, at least 8, at least 9, at least 10, at least 11, or at least twelve 24-hour dietary records. According to other embodiments, the collecting data comprises recording of at least 14, at least 16 or at least eighteen 24-hour dietary records. According to some embodiments, the records are randomly distributed between week and weekend days. This is done to take into account intra-individual variability. The term food comprises everything consumed by a subject including beverages.

According to other embodiments, the collecting data on actual food consumption of a subject comprises at least 7, 9, 11, 12, or fourteen 24-hour dietary records of the consumed food.

According to some embodiments, calculating the Neu5Gc intake comprises evaluating the data from the recorded food consumption based on the measured Neu5Gc content in different types of food. Therefore, prior to calculating Neu5Gc a database of Neu5Gc content in different types of food is formed. Example of such database is presented in Table 2. For each country and/or region, a separated database is used. According to some embodiments, the Neu5Gc content is calculated according to Table 6.

According to some embodiments, determining the antibody repertoire score is perform by a method according to any one of the above embodiments and aspects. According to some embodiments, the antibody repertoire score of above 0.8 or above 0.9 is predictive of increased risk of the disease. According to some embodiments, the antibody repertoire score of above 0.8 or above 0.9 is correlative to increased likelihood of developing a Neu5Gc related disease or disorder.

According to some embodiments, measuring the levels of anti-Neu5Gc antibodies is effected by a method selected from ELISA inhibition assay against a collection or set of Neu5Gc-glycoproteins, ELISA against a collection or set of Neu5Gc-glycopeptides and a collection or set of Neu5Gc-glycans e.g. on a microarray. According to some embodiments, the Neu5Gc associated disease, disorder or condition is selected from atherosclerotic cardiac disease, carcinomas and/or other cancers, and chronic inflammatory conditions.

According to some embodiments, the control subjects are subjects consuming food with low content of Neu5Gc. According to some embodiments, increase of 1.5, 2, 3, 4, 5 times in the ratio between antibodies against Neu5Gcα2-3-linked glycans and antibodies against Neu5Gcα2-6-linked glycans (anti-Neu5Gc α3:α6 ratio) is predictive for developing the disease or disorder. According to some embodiments, the anti-Neu5Gc α3:α6 ratio of above 0.8 is predictive of increased likelihood or increase risk of developing the disease or disorder. According to another embodiment, the anti-Neu5Gc α3:α6 ratio of above 0.9, 1.0, 1.1, or 1.2 is predictive of increased risk or likelihood of developing the disease or disorder. According to another embodiment, the anti-Neu5Gc α3:α6 ratio of above 0.9 is predictive of increased risk or likelihood of developing the disease or disorder. According to another embodiment, the anti-Neu5Gc α3:α6 ratio of above 1.3, 1.4 or 1.5 is predictive of increased risk or likelihood of developing the disease or disorder. According to yet another embodiment, the anti-Neu5Gc a3:a6 ratio of above 1.8, 2, 2.5 or 3 predictive of increased risk or likelihood of developing the disease or disorder.

According to some embodiments, the control is a group of people having low likelihood developing Neu5Gc related disease or disorder. According to one embodiment, the control have anti-Neu5Gc a3:a6 ratio of below 1.2, 1.1, 1, 0.8, 0.79, below 0.75, below 0.7, below 0.65 or below 0.6.

The term "antibody repertoire score" and "anti-Neu5Gc a3:a6 ratio" may be used interchangeably.

According to some embodiments, the method further comprising measuring the level of total anti-Neu5Gc antibodies and incorporating the results in calculation of the likelihood.

According to some embodiments, the anti-Neu5Gc a3:a6 ratio of above 0.8 is predictive of increased likelihood of developing the disease or disorder. According to another embodiment, the anti-Neu5Gc α3:α6 ratio of above 0.9, 1.0, 1.1 or 1.2 is predictive of increased likelihood of developing the disease or disorder. According to yet another embodiment, the anti-Neu5Gc a3:a6 ratio of above 1.8, 2, 2.5 or 3 predictive of increased likelihood of developing the disease or disorder.

The term "likelihood" may have the quantitative and qualitative likelihood.

The terms "comprising", "comprise(s)", "include(s)", "having", "has" and "contain(s)," are used herein interchangeably and have the meaning of "consisting at least in part of". When interpreting each statement in this specification that includes the term "comprising", features other than that or those prefaced by the term may also be present. Related terms such as "comprise" and "comprises" are to be interpreted in the same manner. The terms "have", "has", having" and "comprising" may also encompass the meaning of "consisting of" and "consisting essentially of', and may be substituted by these terms. The term "consisting of" excludes any component, step or procedure not specifically delineated or listed. The term "consisting essentially of" means that the composition or component may include additional ingredients, but only if the additional ingredients do not materially alter the basic and novel characteristics of the claimed compositions or methods.

The terms such as below, above, up to, less than include also the stated value.

As used herein, the term "about", when referring to a measurable value such as an amount, a temporal duration, and the like, is meant to encompass variations of +/-10%, or +/-5%, +/-1%, or even +/-0.1% from the specified value.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Having now generally described the invention, the same will be more readily understood through reference to the following examples, which are provided by way of illustration and are not intended to be limiting of the present invention.

### EXAMPLES

### Methods

### NutriNet-Santé cohort

To investigate the relationship between dietary-Neu5Gc and circulating anti-Neu5Gc antibodies in humans, we used the well-established NutriNet-Santé cohort (ClinicalTrials.gov # NCT03335644). This is a French web-based cohort study launched in 2009 with the objective to investigate the relationship between nutrition (nutrients, foods, dietary patterns, physical activity), health and diseases (e.g. cancer, cardiovascular diseases, metabolic syndrome, rheumatoid arthritis and hypertension), as well as determinants of dietary behaviors and nutritional status. At baseline and every six months, participants completed three non-consecutive validated web-based 24-h dietary records, randomly distributed between week and weekend days to take into account intra-individual variability, in which they declared all foods and beverages consumed during periods of 24 hours. Mean dietary intakes from all the 24h-dietary records available during each participant's follow-up were averaged and considered as usual dietary intakes in these analyses. The NutriNet-Santé web-based self-administered 24h-dietary records have been tested and validated against an interview by a trained dietitian, and against blood and urinary biomarkers. Participants used the dedicated web interface to declare all foods and beverages consumed during a 24h-period for each of the three main meals (breakfast, lunch, dinner) and any other eating occasion, with an accurate estimation of portion sizes. Dietary underreporting was identified on the basis of the method proposed by Black (Int J Obes Relat Metab Disord. 2000;24: 1119-1130), using the basal metabolic rate and Goldberg cut-off, and under-energy reporters (20.0% of the participants of the cohort) were excluded. A subsample of 19,621 volunteer participants attended clinical consultations (69 sites throughout France), where blood samples were collected by trained technicians using a standardized protocol, to constitute the NutriNet-Santé biobank. Of those, we selected individuals with at least six dietary records (16,149 individuals), for which quartiles of Neu5Gc daily intake were calculated. For serum sample detailed analysis, 120 individuals with at least 18 dietary records were selected and included 10 men and 10 women aged 45-60 per Neu5Gc-intake quartile by gender (Q1 - Q4; 80 samples), and 10 men and 10 women aged >60 per quartile, from the first and forth quartiles by gender (Q1 and Q4; 40 samples) (Fig. 1). Men and women were matched for age, education levels and smoking habits.

### Homogenization of food samples.

Samples typically food consumed by the participants of NutriNet-Santé cohort **(Table 2)** were frozen stored in -80 °C. Samples were thawed, 50 mg of each food sample weighed, incubated at -80 °C for 2 hours, then lyophilized overnight. Dried samples were dissolved in 1 ml lysis buffer (50 mM TRIS-HCl pH 7.4, 5 mM MgCl₂, 1 mM Dithiothreitol, 1 mM phenylmethylsulfonyl fluoride), thoroughly vortexed for 30 seconds, put on ice, then sonicated with a probe sonicator (Sonic dismembrator, Fisher scientific) three times at a medium power, each for 10 seconds with 30-seconds intervals incubation on ice. Sonicated solutions were then inserted into a glass dounce tissue grinder (2 ml; Sigma) and homogenized with a loose pestle then with a tight pestle (10 times each). The homogenate was centrifuged 10,000 × g for 5 minutes (min) to remove pelleted nuclei and cell debris, and protein content in the supernatant homogenate was evaluated by a standard BCA assay according to manufactures' protocol (Pierce). The homogenate was stored in -20 °C until use.

### Sialic acid analysis by DMB-HPLC

Sialic acid (Sias) content in food homogenate samples was analyzed as described, with some modifications. Sias were released from glycoconjugates by acid hydrolysis with 0.1 M of H₂SO₄ for 1.5 hours (neutralized with 0.1 M of NaOH) at 80 °C. Free Sias were derivatized with 1,2-diamino-4,5-methylenedioxybenzene (DMB; Sigma) for 2.5 hours at 50 °C, separated by Microcon-10 centrifugal filters and analyzed by fluorescence detection on reverse-phase high pressure liquid chromatography (DMB-HPLC) (Hitachi HPLC Chromaster). HPLC run was on C18 column (Phenomenex C18 Gemini 250 × 4.6 mm) at 24 °C in running buffer (84.5% ddH₂O, 8.5% acetonitrile, 7% methanol) for 60 min at a flow rate of 0.9 ml/min. Quantification of Sias was done by comparison with known quantities of DMB-derivatized Neu5Ac.

### Calculating individual's daily Neu5Gc intake.

We used 19,621 participants enrolled between May 2009 through May 2015 in the NutriNet-Santé Study, and the total amount of dietary Neu5Gc intake (µmole/day) was computed for each participant based on 24 h-dietary records, and measurements of Neu5Gc content in common French food items by DMB-HPLC. The total amount of dietary Neu5Gc intake was computed for each participant based on its consumption of food sources of Neu5Gc estimated using all the available 24-h dietary records and the composition in Neu5Gc of these sources obtained using DMB-HPLC. These food sources of Neu5Gc included meat (cow, lamb, goat, pig, rabbit, and bush meats) and dairy (cow, sheep, buffalo, and goat).

### Measurements of anti-Neu5Gc IgG reactivity by Enzyme-linked immunosorbent assays (ELISA).

Serum anti-Neu5Gc IgG reactivity was measure by three ELISA methods: (1) by an ELISA inhibition assay (EIA assay) using coated wild-type (WT) mouse serum sialo-glycoproteins with *Cmah*^{*-*/*-*} as an adsorbent for non-specific reactivity, as described by Padler-Karavani (PLoS One. 2013;8: e58443); (2) by an ELISA using coated mouse serum glycopepetides (GP assay), as described by Bashir et al., (Bioconjug Chem. 2018, 30(1): 161-168); (3) by an ELISA inhibition assay using coated WT mouse serum sialo-glycopepetides, with the same target as a competitive inhibitor, then deducting the inhibited signal value from the native signal obtained without it (GP-EIA assay), as described by Bashir et al.

### ELISA Inhibition Assay (EIA).

Specific overall anti-Neu5Gc IgGs reactivity in human sera was evaluated by an ELISA against coated mouse serum sialo-glycoproteins, as described in Padler-Karavani (PLoS One. 2013;8: e58443). Briefly, Costar 96-well were coated overnight at 4 °C with 1 µg/well WT pooled mouse sera (lacking mouse-anti-human IgG) in coating buffer (50 mM sodium carbonate-bicarbonate buffer, pH 9.5). Wells were blocked for 2 hours at room temperature (RT) with PBS/OVA blocking buffer (PBS pH 7.3, 1% chicken ovalbumin). During the blocking, human serum was diluted 1: 100 in EIA buffer (PBS pH 7.3, 1% chicken ovalbumin and *Cmah^{-l-}* pooled sera that lacks mouse-anti-human reactivity, diluted at 1:4,000) and incubated on ice for 2 hours. Next, PBS/OVA was removed from wells and pre-incubated human serum was added to triplicate wells at 100 µl/well then incubated at RT for 2 hours. Wells were washed three times with PBST (PBS pH 7.3, 0.1% Tween-20), detection antibody was then added (100 µl/well, 1:7,000 HRP-goat-anti-human IgG diluted in PBS) and incubated for 1 hour at RT. After washing three times with PBST, wells were developed with 0.5 mg/ml O-phenylenediamine in citrate-PO₄ buffer, pH 5.5, reaction was stopped with H₂SO₄ and absorbance was measured at a 490 nm wavelength on a SpectraMax M3 (Molecular Devices).

### Glycopeptides ELISA (GP assay)

Anti-Neu5Gc IgG reactivity in human sera samples was evaluated against coated WT mouse serum glycopeptides by ELISA. Neu5Gc-positive glycopeptides (GP) were prepared from serum of WT C57BL/6 mice, as described in Bashir et al. Costar 96-well were coated overnight at 4 °C with 150 pmol/well GP in coating buffer (50 mM sodium carbonate-bicarbonate buffer, pH 9.5). Wells were blocked for 2 hours at RT with PBS/OVA. After removal of buffer, 1: 100 diluted human sera in PBS/OVA were added to triplicate wells at 100 µl/well then incubated at RT for 2 hours. Wells were washed three times with PBST, detection antibody was then added (100 µl/well, 1:7,000 HRP-goat-anti-human IgG diluted in PBS) and incubated for 1 hour at RT. After washing three times with PBST, wells were developed with 0.5 mg/ml O-phenylenediamine in citrate-PO₄ buffer, pH 5.5, reaction was stopped with H₂SO₄ and absorbance was measured at a 490 nm wavelength on a SpectraMax M3 (Molecular Devices).

### Glycopeptides ELISA Inhibition Assay (GP-EIA assay)

Specific anti-Neu5Gc IgG reactivity in human sera samples was evaluated against coated WT mouse serum glycopeptides compared to competition with the same coated targets by ELISA (GP-EIA assay), as described in Bashir et al. Costar 96-well were coated overnight at 4 °C with GP at 150 pmol Sia/well in coating buffer (50 mM sodium carbonate-bicarbonate buffer, pH 9.5). Wells were blocked for 2 hours at RT with PBS/OVA. During the blocking, human serum was diluted 1: 100 in blocking buffer GP-EIA inhibition buffer (PBS pH 7.3, 1% chicken ovalbumin, GP at 0.03 mMNeu5Gc) and incubated on ice for 2 hours. Next, PBS/OVA was removed from wells and inhibited human serum was added to triplicate wells at 100 µl/well then incubated at RT for 2 hours. Subsequently, wells were further processed as described (GP/EIA assay). To calculate specific anti-Neu5Gc IgG reactivity, the binding signal obtained with GP inhibitor was deducted from the signal obtained against coated GP without the inhibition

### Affinity purification of anti-Neu5Gc antibodies from human sera

Antibodies were affinity-purified from pooled human sera samples (per Neu5Gc-consumption quartile, described in context) on sequential columns of human and chimpanzee serum glycoproteins, as described in Leviatan Ben-Arye, (J. Vis Exp. 2017;125).

### Sialoglycan microarray analysis

Microarrays were fabricated with NanoPrint LM-60 Microarray Printer (Arrayit, CA) on epoxide-derivatized slides (Corning or PolyAn) with 16 sub-array blocks on each slide (Version 3). The list of glycans printed on glycan microarray and their characteristics is presented in **Table** 1. Slides were developed with the selected 120 human serum samples diluted 1:100, detected by Cy3-anti-human IgG (H+L) and analyzed. Briefly, slides were rehydrated with dH₂O and incubated for 30 min in a staining dish with 50 °C pre-warmed 0.05 Methanolamine in 0.1 M of Tris-HCl, pH 9.0 to block the remaining reactive epoxy groups on the slide surface, then washed with 50 °C pre-warmed dH₂O. Slides were centrifuged at 200 × g for 3 min, then fitted with ProPlate^{™} Multi-Array 16-well slide module (Invitrogen) to divide into the 16 subarrays (blocks). Slides were washed with PBST (PBS pH 7.4, 0.1% Tween-20), aspirated, and blocked with 200 µl/sub-array of PBS/OVA blocking buffer for 1 hour at RT with gentle shaking. Next, the blocking solution was aspirated and 100 µl/ block of human serum diluted 1:100 in PBS/OVA was added, then slides were incubated at RT with gentle shaking for 2 hours. Slides were washed three times with PBST then with PBS for 5 min/wash with shaking, then binding detected with 1.5 µg/ml Cy3-goat-anti-human IgG diluted in PBS at 200 µl/block, then incubated at RT for 1 hour. Slides were washed three times with PBST, then with PBS for 5 min/wash followed by removal from ProPlate^{™} Multi-Array slide module which were immediately dipped in a staining dish with dH₂O and were incubated for 10 min with shaking followed by centrifugation at 200 × g for 5 min, then dry slides scanned immediately.

**Table 1. List of glycans printed on glycan microarray and their characteristics.**

| **Glycan ID** | **Structure** | **Sialic Acid** | | **Skeleton** | **Pairs of Neu5Gc/ Neu5Ac glycans** |
|---|---|---|---|---|---|
| | | **Type** | **Linkage** | | |
| 1 | Neu5,9Ac₂α3Galβ4GlcNAcβ O(CH₂)₃NH₂ | Neu5,9Ac₂ | α3 | LacNAc | P1-Ac |
| 2 | Neu5Gc9Acα3Galβ4GlcNAc βO(CH₂)₃NH₂ | Neu5Gc9Ac | α3 | LacNAc | P1-Gc |
| 3 | Neu5,9Ac₂α6Galβ4G1cNAcβ O(CH₂)₃NH₂ | Neu5,9Ac₂ | α6 | LacNAc | P2-Ac |
| 4 | Neu5Gc9Acα6Galβ4GlcNAc βO(CH₂)₃NH₂ | Neu5Gc9Ac | α6 | LacNAc | P2-Gc |
| 5 | Neu5Acα6GalNAcαO(CH₂)₃ NH₂ | Neu5Ac | α6 | GalNAc | P3-Ac |
| 6 | Neu5Gcα6GalNAcαO(CH₂)₃ NH₂ | Neu5Gc | α6 | GalNAc | P3-Gc |
| 7 | Neu5,9Ac₂α3Galβ3GlcNAcβ O(CH₂)₃NH₂ | Neu5,9Ac₂ | α3 | Type 1 | P4-Ac |
| 8 | Neu5Gc9Acα3Galβ3GlcNAc βO(CH₂)₃NH₂ | Neu5Gc9Ac | α3 | Type 1 | P4-Gc |
| 9 | Neu5,9Ac₂α3Galβ3GalNAcα O(CH₂)₃NH₂ | Neu5,9Ac₂ | α3 | Core 1 | PS-Ac |
| 10 | Neu5Gc9Acα3Galβ3GalNAc αO(CH₂)₃NH₂NH₂ | Neu5Gc9Ac | α3 | Core 1 | P5-Gc |
| 11 | Neu5Acα3Galβ4GlcNAcβO( CH₂)₃NH₂ | Neu5Ac | α3 | LacNAc | P6-Ac |
| 12 | Neu5Gcα3Galβ4GlcNAcβO( CH₂)₃NH₂ | Neu5Gc | α3 | LacNAc | P6-Gc |
| 13 | Neu5Acα3Galβ3GlcNAcβO( CH₂)₃NH₂ | Neu5Ac | α3 | Type 1 | P7-Ac |
| 14 | Neu5Gcα3Galβ3GlcNAcβO( CH₂)₃NH₂ | Neu5Gc | α3 | Type 1 | P7-Gc |
| 15 | Neu5Acα3Galβ3GalNAcαO( CH₂)₃NH₂ | Neu5Ac | α3 | Core 1 | P8-Ac |
| 16 | Neu5Gcα3Galβ3GalNAcαO( CH₂)₃NH₂ | Neu5Gc | α3 | Core 1 | P8-Gc |
| 17 | Neu5Acα6Galβ4GlcNAcβO( CH₂)₃NH₂ | Neu5Ac | α6 | LacNAc | P9-Ac |
| 18 | Neu5Gcα6Galβ4GlcNAcβO( CH₂)₃NH₂ | Neu5Gc | α6 | LacNAc | P9-Gc |
| 19 | Neu5Acα6Galβ4GlcβO(CH₂) ₃NH₂ | Neu5Ac | α6 | Lactose | P10-Ac |
| 20 | Neu5Gcα6Galβ4GlcβO(CH₂) ₃NH₂ | Neu5Gc | α6 | Lactose | P10-Gc |
| 21 | Neu5Acα3Galβ4GlcβO(CH₂) ₃NH₂ | Neu5Ac | α3 | Lactose | P11-Ac |
| 22 | Neu5Gcα3Galβ4GlcβO(CH₂) ₃NH₂ | Neu5Gc | α3 | Lactose | P11-Gc |
| 23 | Neu5,9Ac₂α6GalNAcαO(CH₂ )₃NH₂ | Neu5,9Ac₂ | α6 | GalNAc | P 12-Ac |
| 24 | Neu5Gc9Aca6GalNAcaO(C H₂)₃NH₂ | Neu5Gc9Ac | α6 | GalNAc | P 12-Gc |
| 25 | Neu5Acα3GalβO(CH₂)₃NH₂ | Neu5Ac | α3 | Galactose | P13-Ac |
| 26 | Neu5Gcα3GalβO(CH₂)₃NH₂ | Neu5Gc | α3 | Galactose | P13-Gc |
| 27 | Neu5Acα6GalβO(CH₂)₃NH₂ | Neu5Ac | α6 | Galactose | P14-Ac |
| 28 | Neu5Gcα6GalβO(CH₂)₃NH₂ | Neu5Gc | α6 | Galactose | P14-Ac |
| 29 | Neu5,9Ac₂α3GalβO(CH₂)₃N H2 | Neu5,9Ac₂ | α3 | Galactose | P15-Gc |
| 30 | Neu5Gc9Acα3GalβO(CH₂)₃ NH₂ | Neu5Gc9Ac | α3 | Galactose | P15-Ac |
| 31 | Neu5,9Ac₂α6GalβO(CH₂)₃N H₂ | Neu5,9Ac₂ | α6 | Galactose | P16-Gc |
| 32 | Neu5Gc9Acα6GalβO(CH₂)₃ NH₂ | Neu5Gc9Ac | α6 | Galactose | P16-Ac |
| 33 | Neu5Acα3Galβ3GalNAcβO( CH₂)₃NH₂ | Neu5Ac | α3 | Core 1 | P17-Gc |
| 34 | Neu5Gcα3Galβ3GalNAcβO( CH₂)₃NH₂ | Neu5Gc | α3 | Core 1 | P17-Ac |
| 35 | Neu5,9Ac₂α3Galβ3GalNAcβ O(CH₂)₃NH₂ | Neu5,9Ac₂ | α3 | Core 1 | P18-Gc |
| 36 | Neu5Gc9Acα3Galβ3GalNAc βO(CH₂)₃NH₂ | Neu5Gc9Ac | α3 | Core 1 | P18-Ac |
| 37 | Neu5,9Ac₂α6Galβ4GlcβO(C H₂)₃NH₂ | Neu5,9Ac₂ | α6 | Lactose | P19-Gc |
| 38 | Neu5Gc9Ac6Galβ4GlcβO(C H₂)₃NH₂ | Neu5Gc9Ac | α6 | Lactose | P19-Ac |
| 39 | Neu5,9Ac₂α3Galβ4GlcβO(C H₂)₃NH₂ | Neu5,9Ac₂ | α3 | Lactose | P20-Ac |
| 40 | Neu5Gc9Ac3Galβ4GlcβO(C H₂)₃NH₂ | Neu5Gc9Ac | α3 | Lactose | P20-Gc |
| 41 | Neu5Acα8Neu5Acα3Galβ4G lcβO(CH₂)₃NH₂ | Neu5Ac-Neu5Ac | α3-α8 | Lactose | |
| 42 | Neu5Acα8Neu5Acα8Neu5Ac α3Galβ4GlcβO(CH₂)₃NH₂ | (Neu5Ac)₃ | α3-α8 | Lactose | |
| 55 | Neu5Acα3Galβ4(Fucα3)Glc NAcβO(CH₂)₃NH₂ | Neu5Ac | α3 | Le^{x} | P21-Ac |
| 56 | Neu5Gcα3Galβ4(Fucα3)Glc NAcβO(CH₂)₃NH₂ | Neu5Gc | α3 | Le^{x} | P21-Gc |
| 57 | Neu5Acα3Galβ4(Fucα3)Glc NAc6SβO(CH₂)₃NH₂ | Neu5Ac | α3 | 6S-Le^{x} | P22-Ac |
| 58 | Neu5Gcα3Galβ4(Fucα3)Glc NAc6SβO(CH₂)₃NH₂ | Neu5Gc | α3 | 6S-Le^{x} | P22-Gc |
| 60 | Neu5Acα3Galβ3GlcNAcβ3G alβ4GlcβO(CH₂)₃NH₂ | Neu5Ac | α3 | LNT | P23-Ac |
| 61 | Neu5Gcα3GalβGlcNAcβG alβ4GlcβO(CH₂)₃NH₂ | Neu5Gc | α3 | LNT | P23-Gc |
| 62 | Neu5Acα3Galβ4GlcNAc6Sβ O(CH₂)₃NH₂ | Neu5Ac | α3 | 6S-LacNAc | P24-Ac |
| 63 | Neu5Gcα3Galβ4GlcNAc6Sβ O(CH₂)₃NH₂ | Neu5Gc | α3 | 6S-LacNAc | P24-Gc |
| 67 | Neu5Acα6(Neu5Gcα3)Galβ4 GlcβO(CH₂)₃NH₂ | Neu5Ac/ Neu5Gc | α3/α6 | Lactose | |
| 69 | Neu5Gcα8Neu5Acα3Galβ4G lcβO(CH₂)₃NH₂ | Neu5Gc-Neu5Ac | α3-α8 | Lactose | |
| 72 | Neu5Acα8Neu5Gcα3Galβ4G lcβO(CH₂)₃NH₂ | Neu5Ac-Neu5Gc | α3-α8 | Lactose | |
| 73 | Neu5Acα8Neu5Gcα6Galβ4G lcβO(CH₂)₃NH₂ | Neu5Ac-Neu5Gc | α6-α8 | Lactose | |
| 75 | Neu5 Gcα8Neu5Gcα3Gal(34G lcβO(CH₂)₃NH₂ | Neu5Gc-Neu5Gc | α3-α8 | Lactose | |
| 78 | Galα3Galβ4GlcNAβO(CH₂) ₃NH₂ | N/A | N/A | LacNAc | αGal |

Sia-linkages (Siaa2-3/6/8 linkages; α3, α6, α8, respectively) or underlying skeleton glycans [Lac (lactose; Galβ3Glc), Gal (galactose), Type 1 (Galβ3GlcNAc), GalNAc (N-acetylgalactoseamine), LacNAc (lactoseamine; Galβ4GlcNAc), Core 1 (Galβ3GalNAc)].

### Array slide processing

Processed slides were scanned and analyzed at 10 µm resolution with a Genepix 4000B microarray scanner (Molecular Devices) using 350 gain, as described Images were analyzed by Genepix Pro 6.0 software (Molecular Devices). Spots were defined as circular features with a variable radius and local background subtraction was performed. Data were analyzed by Excel.

### Affinity equilibrium constant K_{D} calculation by microarray

The affinity of serum anti-Neu5Gc IgG against diverse Neu5Gc-glycans was analyzed by glycan microarray. Briefly, slides were developed as described above with serial dilutions (a factor of 2) of affinity-purified pooled serum anti-Neu5Gc IgG antibodies ranging at 40 ng/µl - 6.1 × 10⁻⁴ ng/µl (266.67 nM - 0.033 nM) in PBS/OVA blocking buffer. K_{D} was calculated by fitting a plot of response at equilibrium against a wide range of purified anti-Neu5Gc antibody concentrations (non-linear fit with one-site specific binding, GraphPad Prism 7.0).

### Statistical analyses

As nutritional habits and intakes vary across gender, we computed statistical analyses by gender. Apart from total Neu5Gc intakes, three additional classes of dietary Neu5Gc sources were computed: Neu5Gc from meat, Neu5Gc from dairy cow and Neu5Gc from dairy sheep and goat. Sex-specific tertiles for dietary Neu5Gc intakes were computed for each class (total daily Neu5Gc intake, daily Neu5Gc intake from meat, from dairy cow, and from dairy sheep and goat combined).

Statistical analyses performed by SAS 9.4^{®}. Quantitative variables presented as means ± standard deviation (SD) or means ± standard error of the mean (sem) if normally distributed, or as medians (25th - 75th percentiles) if not normally distributed, while qualitative variables were presented as percentages. Mann-Whitney U, median tests or ANOVA allowed to compare means and medians between two groups, or more than two groups, of quantitative variables, while chi square test examined significant differences across two qualitative variables. We also studied the associations between variables derived from antibodies measures (individual Neu5Gc- and Neu5Ac- glycans, linear combinations of Neu5Gc glycans, and αGal), and tertiles of Neu5Gc intake (Neu5Gc sources in four variables: total, cow meat, dairy cow and dairy sheep and goat) or Neu5Ac using the SAS^{®} quantreg procedure (a non-parametric regression that compares the median values of the outcome variables across the tertiles of the predictive variables). We used median regression because Neu5Gc intakes were not normally distributed. All the tests were two sided and the statistical significance set at 0.05.

### Example 1. Evaluating levels of daily Neu5Gc intake from red meat and dairy

The amounts of Neu5Ac and Neu5Gc were quantitated in the diverse food items **(Table 2).** On average, Neu5Ac content was ~3 times greater than Neu5Gc (414 ± 58 nmol/gr versus 149 ± 30 nmol/gr, respectively; mean ± sem). Neu5Gc content was highest in dairy sheep and goat products, moderate in red meat, but rather low in dairy cow products (422 ± 10 nmol/gr, 118 ± 17 nmol/gr, 21 ± 1 nmol/gr, respectively). Yet in practice, daily dietary Neu5Gc intake relies on actual amounts of food consumed by individuals (e.g. common red meat serving size is ~250 gr/day, while much lower for dairy).

**Table 2. Sialic acids content (Neu5Ac and Neu5Gc) in common French food items measured by DMB-HPLC.**

| **Source** | **Food item** | **Sialic acid type** | |
|---|---|---|---|
| | | **Neu 5 Ac** | **Neu5Gc** |
| | | **Average ± sem (nmol/gr)** | **Average ± sem (nmol/gr)** |
| Dairy | | | |
| Buffalo | Mozzarella | 138 ± 43 | 6± 1 |
| Cow | Parmesan | 842 ± 94 | 11 ± 2 |
| | Crème fraiche | 501 ± 126 | 11 ± 4 |
| | Gouda | 562 ± 169 | 18 ± 6 |
| | Plain yogurt | 515 ± 163 | 18 ± 7 |
| | Petit suisse | 461 ± 222 | 18 ± 9 |
| | Powdered milk | 1847 ± 279 | 107 ± 36 |
| | Milk | 781 ± 101 | 21 ± 4 |
| | Cheese strainer | 557 ± 141 | 21 ± 8 |
| | Fromage blanc | 549 ± 91 | 23 ± 11 |
| | Cheese spread | 664 ± 145 | 23 ± 13 |
| | Camembert | 987 ± 144 | 59 ± 7 |
| Goat | Feta | 239 ± 11 | 280 ± 15 |
| | Soft goat cheese | 278 ± 49 | 336 ± 62 |
| | Roll of goat cheese | 431 ± 120 | 344 ± 104 |
| | Dry cheese | 399 ± 138 | 533 ± 211 |
| Sheep | Yogurt | 153 ±46 | 277 ± 85 |
| | Roquefort | 49 ± 2 | 550 ± 13 |
| | Etorki | 81 ± 8 | 633 ± 78 |

| Red and processed meat | | | |
|---|---|---|---|
| Cow | Dried sausage | 292 ± 60 | 22 ± 9 |
| | Chipolatas sausages | 395 ± 252 | 32 ± 3 |
| | Inards | 418 ± 142 | 110 ± 65 |
| | Beef tongue | 257 ± 123 | 131 ± 124 |
| | Beef | 234 ± 33 | 163 ± 17 |
| | Pate | 196 ± 85 | 307 ±240 |
| Pig | Pork | 90 ± 12 | 18 ± 3 |
| | Bacon strips | 368 ± 215 | 31 ± 15 |
| | Pork rilettes | 122 ± 21 | 68 ± 41 |
| | Ham | 349 ± 239 | 71 ± 48 |
| | Cured ham | 232 ± 49 | 140 ± 89 |
| | Strasbourg sausage | 121 ± 72 | 207 ± 174 |
| | Liver | 473 ± 107 | 409 ± 63 |
| Lamb | | 324 ± 224 | 67 ± 58 |
| Rabbit | | 164 ± 73 | 7 ± 4 |

### Example 2. Assessment of daily Neu5Gc intake

To account for individual records, daily Neu5Gc intake was calculated from all available NutriNet-Santé participants enrolled between May 2009 through May 2015, and that had a minimum of six 24-h dietary records (16,149 participants of 19,621 registered; Fig. 1). Based on these questionnaires and food Neu5Gc measurements, daily Neu5Gc intake was calculated per participant, then quartiles of total dietary Neu5Gc intakes were computed by gender (Q1-Q4; **Table 3,** **Fig. 1****,** and **Fig. 2**).

**Table 3. Quartiles of daily Neu5Gc intake in NutriNet-Santé participants (May 2009 through May 2015) with a minimum of six 24-hour dietary records (total 16,149 participants).**

| **Group** | **N** | **Daily Neu5Gc intake (µmol/day) (calculated from ≥ 6 24h-dietary records)** | | | |
|---|---|---|---|---|---|
| | | **Min** | **Mean** | **Median** | **Max** |
| By quartile | | | | | |
| Q1 | 4037 | 0 | 8.2 | 8.9 | 11.5 |
| Q2 | 4037 | 11.5 | 13.6 | 13.7 | 15.7 |
| Q3 | 4038 | 15.7 | 18 | 17.9 | 20.9 |
| Q4 | 4037 | 20.9 | 28.4 | 25.6 | 181.5 |

| By gender | | | | | |
|---|---|---|---|---|---|
| Men | 4562 | 0 | 19.7 | 18.4 | 167.6 |
| Women | 11587 | 0 | 16.1 | 14.8 | 181.5 |

| By age and gender (Men) | | | | | |
|---|---|---|---|---|---|
| 18-30 y | 216 | 0 | 17.6 | 16.5 | 66.8 |
| 31-44 y | 588 | 0 | 18 | 16.6 | 85.9 |
| 45-60 y | 1240 | 0.1 | 20 | 18.9 | 83.2 |
| > 60 y | 2518 | 0 | 20.1 | 18.7 | 167.6 |

| By age and gender (Women) | | | | | |
|---|---|---|---|---|---|
| 18-30 y | 1006 | 0 | 13.6 | 13.1 | 64 |
| 31-44 y | 2040 | 0 | 15 | 14 | 93.3 |
| 45-60 y | 4779 | 0 | 16.2 | 14.9 | 104.7 |
| > 60 y | 3762 | 0 | 17.1 | 15.6 | 181.5 |

### Example 3. Anti-Neu5Gc antibodies measurement in 120 representative individuals

For further detailed analysis, 120 representative individuals who provided at least 18 dietary records, and had available blood samples, were randomly selected **(Table 4).**

**Table 4. General characteristics of the study cohort of 120 representative individuals, each with at least 18 24h-dietary records**

| **Age group:** | **45-60** | | | **> 60** | | |
|---|---|---|---|---|---|---|
| **Characteristics** | **Men** | **women** | **P** | **Men** | **women** | **P** |
| N | 40 | 40 | | 20 | 20 | |
| Age, in years | 57.0 ± 4.5 | 58.0 ± 4.4 | 0.27 | 70.3 ± 4.6 | 69.2 ± 4.7 | 0.45 |

| Educational level: | | | | | | |
|---|---|---|---|---|---|---|
| Primary or less | 0 | 5 | 0.44 | 5 | 15 | 0.55 |
| Secondary | 37.5 | 42.5 | | 30 | 30 | |
| University | 62.5 | 52.5 | | 65 | 55 | |

| Tobacco smoking: | | | | | | |
|---|---|---|---|---|---|---|
| Non smokers | 47.5 | 47.5 | 0.6 | 30 | 45 | 0.28 |
| Ex-smokers | 37.5 | 45 | | 60 | 35 | |
| Current smokers | 15 | 7.5 | | 10 | 20 | |
| Energy intake, Kcal/day | 2411 ± 450 | 1774 ± 336 | 0.0001 | 2247 ± 387 | 1780 ± 252 | 0.0001 |
| Proteins intake, g/day | 94.1 ± 18.6 | 74.1 ± 16.9 | 0.0001 | 93.3 ± 18.4 | 74.0 ± 12.5 | 0.0004 |
| Animal protein intake, g/day | 62.1 ± 16.7 | 49.3 ± 16.0 | 0.0008 | 62.3 ± 18.1 | 49.5 ± 11.5 | 0.01 |
| Lipids intakes, g/day | 102.3 ± 23.0 | 74.9 ± 16.3 | 0.0001 | 92.6 ± 20.3 | 80.3 ± 18.1 | 0.051 |
| Carbohydrates intakes, g/day | 252.5 ± 64.8 | 189.6 ± 46.1 | 0.0001 | 226.2 ± 56.6 | 174.9 ± 33.9 | 0.001 |
| Number of 24-hour dietary records | 21.4 ± 2.9 | 21.6 ± 3.3 | 0.83 | 21.7 ± 3.2 | 22.9 ± 3.4 | 0.26 |

| | | | | | | |
|---|---|---|---|---|---|---|
| means ± SD or median of 25^{th} - 75^{th} percentiles for continuous variables; relative frequencies for qualitative variables. | | | | | | |

This focused/selected cohort included 10 men and 10 women aged 45-60 per Neu5Gc-intake quartile by gender (Q1 - Q4; 80 samples), and 10 men and 10 women aged >60 per quartile, from the first and forth quartiles by gender (Q1 and Q4; 40 samples). Selected men and women were matched for age, education levels and smoking habits **(Table 4),** but nutritional habits and intakes were expected to vary across gender. Accordingly, in this cohort there were statistically significant differences between men and women in energy intake, and intake of proteins, animal proteins, lipids and carbohydrates **(Table 4).** Hence, the total daily Neu5Gc intake in this study cohort was first computed by gender and age **(Table 3).** In addition, dietary Neu5Gc was divided into three sub-classes based on the contributing food source (red meat, dairy cow, and dairy sheep or goat). Total daily Neu5Gc intake was significantly higher in men versus women aged 45-60, largely contributed due to higher consumption of red meat. Similar trends were found in the >60 age group, though differences were not statistically significant. There were no significant differences in dairy consumption between men and women.

It is not trivial to measure immune responses against Neu5Gc in light of the fact that a large collection of Neu5Gc-neoantigens appear on diverse glycans, glycoproteins and glycolipids, at different densities and combinatorial collections on cell surfaces. To assess the humoral responses against Neu5Gc, serum samples of the selected cohort were initially analyzed for their specific overall anti-Neu5Gc IgG reactivities. This was done against a collection of multiple Neu5Gc-containing antigens, by three established methods that differ in their target antigens and/or signal measurements: (1) ELISA (enzyme-linked immunosorbent assay) inhibition assay against a collection of Neu5Gc-glycoproteins (2) ELISA against a collection of Neu5Gc-glycopeptides (GP assay); (3) ELISA against a collection of Neu5Gc-glycopeptides with signal calculated after deduction of residual background reactivity (GP-EIA assay) (see materials and methods). Hence, a total of 120 sera samples were quantitavely analysed by EIA, GP and GP-EIA to detect overall anti-Neu5Gc IgG responses (Fig. 4A). Frequency of distribution of the overall anti-Neu5Gc IgG reactivity demonstrated that the sensitivity of EIA was much higher than that of GP or GP-EIA assays (2-4 times higher concentration range, with a mean of distribution of 4.4 ng/µl in EIA, but only 2 ng/µl and 1.1 ng/µl in GP and GP-EIA, respectively **(****Fig. 3A****).** Direct comparison between the assays showed that while GP correlated with GP-EIA (both measured against Neu5Gc-glycpeptides), these two assays had no correlation with EIA **(****Fig. 3B****).** This was despite the fact that the same coated-target glycans were used for serum antibodies binding in all three assays, only that the glycans were conjugated to carrier proteins (glycoproteins; EIA) or to carrier peptides (glycopeptides; GP/GP-EIA). Of note, the Neu5 Gc-glycopeptides were generated by degradation of the protein-carrier of native Neu5Gc-glycoproteins. Hence, in EIA assay the glycans were presented in the context of the native Neu5Gc-glycoproteins, while in GP/GP-EIA there was a much denser population of coated-glycans targets. These differences likely affected the antibody binding in a way that only a fraction of the circulating antibodies was measured by GP/GP-EIA assays. These findings support previous studies that demonstrated the reliability of the EIA assay for measurements of overall anti-Neu5Gc IgG responses. The EIA assay has a wider detection range and can detect the overall anti-Neu5Gc IgG response, while the GP or GP-EIA detect only a fraction of this response. In addition, they highlight the importance of the presentation mode of glycans that can mediate detection of different pools of antibodies within the sera, even against the same glycan targets.

Importantly, EIA overall anti-Neu5Gc IgG responses showed a clear gender difference, with almost twice as much higher levels in men compared to women **(****Fig. 3C****).** The medians in the 45-60 age group were 3.94 µg/ml in men and 2.22 µg/ml in women (95% confidence interval (CI) 2.35 to 5.26 and 95% CI 1.83 to 3.38, respectively) representing a 1.8 fold difference, and in the >60 age group the medians were 4.75 µg/ml in men and 2.47 µg/ml in women (95% CI 2.51 to 6.82 and 95% CI 1.91 to 3.71) representing a 1.9 fold difference. These higher anti-Neu5Gc IgG levels in men compared to women were associated with the similar gender differences in daily Neu5Gc intake.

### Example 4. Analysis of anti-Neu5Gc responses by glycan microarray

To gain further insight into the full characteristics of the responses against Neu5Gc and the diet effects, serum samples of the selected cohort **(**Fig. 2B-C) were analyzed by sialoglycan microarrays. These were fabricated with a diverse collection of Neu5Gc-glycan antigens and their matching pairs of Neu5Ac-glycans, each with a terminal primary amine that mediated their covalent conjugation onto epoxide-coated slides **(Table 1).**

Human serum IgG reactivity against each printed glycan was analyzed and quantified. Total IgG reactivity per donor was assessed as the sum of reactivities against all Neu5Gc-glycans versus all NeuSAc-glycans, and samples divided according to quartiles of total daily Neu5Gc intake **(****Fig. 4A****).** In both men and women aged 45-60, the total reactivities against Neu5Gc-glycans appeared to be higher in elevated Neu5Gc consumption levels, while those against Neu5Ac-glycans were significantly lower and did not change by dietary quartile **(****Fig. 4A****;** p < 0.0001). Similar trends were observed in the group aged >60, although Neu5Ac reactivities appeared to be higher than those of the group aged 45-60. Of note, the reactivity against Neu5Gc-glycans correlated with the reactivity against NeuSAc-glycans only in the group aged >60 of high Neu5Gc consumers (Q4; R²=0.74). The somewhat higher anti-Neu5Ac IgG in individuals >60 likely may represent cross-reactivity of anti-Neu5Gc IgG with NeuSAc-glycans, and fit the notion that the quality of immune responses deteriorates with age.

To further investigate the Neu5Gc dietary effect, we evaluated the immune responses against another immunogenic non-human sugar that shares common features with Neu5Gc. Similar to Neu5Gc, the αGal glycan antigen (Galα1-3Galβ1-4GlcNAc-R) cannot be synthesized by human cells due to a specific deletion in the gene encoding α1,3galactosyltransferase. Yet it is highly expressed by gut microbiota, and as a result all humans express circulating anti-Gal antibodies. αGal is also expressed in mammalian derived food as red meat and dairy, however once consumed it is broken down into native human self-monosaccharides, as galactose. Thus, while both Neu5Gc and αGal are foreign antigens in humans, only Neu5Gc can incorporate into human cells through the diet and generate neoantigens. Given these differences, humoral responses against Neu5Gc-neoantogens are likely to be affected by the diet, in contrast to those against αGal. To investigate this aspect, the αGal antigen was printed side-by-side with the Neu5Ac-/Neu5Gc-glycans on the microarrays and human serum IgG reactivity investigated per Neu5Gc-dietary quartile **(****Fig. 4B****).** The array analysis clearly showed that the anti-Gal reactivity did not change between quartiles in all groups, in contrast to the higher reactivity against Neu5Gc-glcycans associated with elevated consumption of Neu5Gc **(****Fig. 4****).** Altogether, these data clearly demonstrated the high specificity of anti-Neu5Gc IgG responses in human sera. Furthermore, dietary Neu5Gc positively affected anti-Neu5Gc IgG responses, but did not have any effect on the related IgG responses against native NeuSAc-glycans nor the αGal antigenic glycan.

### Example 5. Association between Neu5Gc intake and levels and altered diversity of anti-Neu5Gc IgGs

For detailed assessment of the dietary effects on antibodies characteristics, they were evaluated in individuals aged 45-60 per quartile of total Neu5Gc intake by gender **(****Fig. 5****).** In both men and women total Neu5Gc intake was gradually elevated between quartiles, with much higher levels in Q4 **(****Fig. 5****).** Men consumed almost twice red meat as much as dairy, most obvious in Q4 (**Fig. 5A** **and** **Fig. 6****;** total Neu5Gc intake across quartiles was 56% ± 2.7% for red meat, while 31% ± 2.5% for dairy from cow, and 12.9% ± 2.4% for dairy from sheep & goat; mean% ± sem%). In fact, there was a significant overlap between men divided in quartiles based on total Neu5Gc consumption or based on Neu5Gc from red meat overlap was 63% ± 10%). Women appeared to consume more dairy than meat (44.2% ± 3% red meat, 42.1% ± 3.3% dairy from cow, 13.6% ± 2.4% dairy from sheep & goat; **Fig. 6**), reaching comparable levels in Q4 **(****Fig. 5B****).** Hence, Neu5Gc was dominantly contributed from red meat in men, while dairy in women.

To gain further insight into the effects of higher Neu5Gc intake on antibodies, sera were examined by sialoglycan microarrays against 24 different Neu5Gc-glycans (P1-Gc to P24-Gc), and the sum of anti-Neu5Gc IgG levels assessed per quartile. This detailed analysis revealed that anti-Neu5Gc IgG responses were higher in men compared to women, supporting the results obtained with the EIA assay that estimated overall anti-Neu5Gc IgG responses against diverse natural Neu5Gc-glycoproteins, and also showed significant differences **(****Fig. 3C****).** This correlation between EIA measurements and the sum of the detailed antibody reactivities measured against individual glycans by microarray, suggest that the EIA can provide a reliable assessment of the levels of anti-Neu5Gc IgG. In both men and women, the sum of anti-Neu5Gc IgG was higher in Q2 compared to Q1 and remained high through Q4. The differences in antibody levels were more prominent in men, likely owing to their much higher Neu5Gc intake compared to women. In fact, when men were stratified according to quartiles of Neu5Gc consumption from red meat, their dominantly contributing food source, anti-Neu5Gc IgG levels seemed to be almost twice as much in Q2-Q4 compared to Q1. Similarly, dividing women according to quartiles of Neu5Gc intake from dairy from cow showed higher antibodies levels in Q3-Q4 compared to Q1-Q2. Therefore, despite the difficulties in assessing the direct contribution of each food source on antibody levels, the intrinsic differences between the dietary habits of French men versus women allowed to highlight the notion that higher total Neu5Gc, either from red meat or from dairy, contribute to higher levels of circulating serum anti-Neu5Gc IgG.

To assess the impact of Neu5Gc intake on the repertoire of anti-Neu5Gc IgG, serum IgG reactivities against 24 different Neu5Gc-glycans were stratified according to common features, as linkages to underlying glycans (Siaa2-3/6/8 linkage; a3, α6, α8, respectively), and underlying glycan skeletons (Lac, Gal, Type 1, GalNAc, LacNAc, Core 1; Fig. 5). In both men and women, lower Neu5Gc intake was associated with higher levels of anti-Neu5Gc IgG reactive against Neu5Gcα2-6-linked glycans (α6), while higher Neu5Gc intake was rather associated with reactivities against Neu5Gcα2-3-linked glycans (α3;), see **Table 5.**

**Table 5. Distribution of anti-Neu5Gc antibodies according to glycan linkage**

| | **Women** | | | | | **Men** | | | |
|---|---|---|---|---|---|---|---|---|---|
| **Linkage** | **Q1** | **Q2** | **Q3** | **Q4** | | **Q1** | **Q2** | **Q3** | **Q4** |
| **Sum α3** | 738 | 1099 | 1158 | 1367 | | 888 | 2155 | 1833 | 2032 |
| **Sum α6** | 835 | 1120 | 1154 | 868 | | 1501 | 1446 | 1548 | 1626 |
| **Sum α8** | 17 | 146 | 144 | 53 | | 25 | 61 | 81 | 237 |
| **α3/α6 Ratio** | 0.88 | 0.98 | 1.00 | 1.58 | | 0.59 | 1.49 | 1.18 | 1.25 |

Similar changes were noticed when quartiles were divided according to the dominant food source in men or women. In fact, linkage recognition had gradually shifted from α6 to α3 as Neu5Gc intake increased **(****Fig. 5****).** Similarly, there was a gradual shift in recognition of Neu5Gc-glycans with underlying lactose (Lac) skeleton, and to a lesser extent with underlying Type 1. Altogether, these changes suggest that irrespectively of gender or dominant Neu5Gc food source, higher Neu5Gc intake was associated with altered diversity of anti-Neu5Gc IgGs, specifically shifting towards IgGs recognizing Neu5Gcα2-3-linked glycans, mostly associated with increased underlying lactose skeleton. Of note, both lactose and α3-linkage are features most commonly found with sialic acid containing glycolipids (gangliosides), although they can also be found on glycoproteins.

### Example 7. Anti-Neu5Gc IgG as function of a reason for their generation

To evaluate the specificity of the Neu5Gc-diet related effects on anti-Neu5Gc antibodies levels and diversity, we examined these effects in sera from individuals with known elevated levels of anti-Neu5Gc IgG, but that were not related to diet. It had previously been shown that during acute clinically overt Epstein-Bar Virus (EBV) primo-infection (Infectious mononucleosis; IMN), the levels of anti-Neu5Gc IgG are significantly increased compared to healthy controls matched for age and gender, as measured by EIA assay (Le Berre et al., Clin Immunol. 2017, 180, 128-135). To further evaluate changes in anti-Neu5Gc IgG repertoire in this situation, we obtained samples from the same cohort of 45 IMN patients and from 43 age/gender-matched healthy individuals **(****Fig. 7A****),** and examined those on sialoglycan microarrays **(**Fig. 7B-D). This analysis confirmed the increase in anti-Neu5Gc IgG levels also by microarray **(****Fig. 7B****),** corroborating the previous EIA analysis. In addition, the reactivity was highly specific to Neu5Gc with no recognition of NeuSAc-glycans **(****Fig. 7C****).** Further examination of the repertoire revealed no differences between IMN patients and controls (Fig. 7D), despite the increased levels of antibodies and their high specificity. In fact, the ratios between reactivities against Neu5Gc-glycans with the different linkages (α3, α6, α8) had not changed at all. Since EBV do not harbour the CMAH gene that encodes the enzyme responsible for Neu5Gc synthesis, the possible interpretation of the significant rise in anti-Neu5Gc IgGs was related to the extremely high percentage of B cells randomly infected, including the progenitors with a sIg receptor specific for the EBV. These findings are in clear contrast to the changes in anti-Neu5Gc IgG linkage-diversity observed with increased Neu5Gc related to diet, in which there is more recognition of α3-linkage as levels of Neu5Gc intake increase, replacing the preferred α6-linkage at lower Neu5Gc intake levels **(****Fig. 5****).** Altogether these data provide strong evidence that Neu5Gc from diet can affect the levels and repertoire diversity of circulating serum anti-Neu5Gc antibodies, in both men and women, irrespectively of the dietary source contributing to higher Neu5Gc intake.

### Example 8. Affinity-purification of anti-Neu5Gc antibodies

We have previously shown that human immunization with a drug containing Neu5Gc-glycoproteins (the drug anti-thymocyte globulin; ATG; commonly used as an immunosuppressive treatment) can cause a transient increase in anti-Neu5Gc IgG responses, with an altered repertoire. Such drug-elicited increased anti-Neu5Gc IgG levels were also associated with higher affinities of the developed antibodies at peak of response, some also acquiring new specificities. To examine whether Neu5Gc-diet-induced anti-Neu5Gc IgG also acquire higher affinities, as in the case of drug-induced antibodies, serum anti-Neu5Gc antibodies from Q1 and Q4 by gender and age were affinity-purified. Due to limited volumes of samples, sera were pooled. Men aged 45-60, were divided according to Neu5Gc intake from red meat, then 10 serum samples per quartile were pooled and anti-Neu5Gc antibodies affinity-purified on sequential columns of NeuSAc-glycoproteins and Neu5Gc-glycoproteins, then eluted from the latter with free Neu5Gc. The yield of affinity-purification clearly demonstrated twice as much higher yields in Q4 compared to Q1 **(****Fig. 8A****),** that is in complete agreement of the observed higher levels and reactivities found by serum analysis. Moreover, the purified antibodies were highly specific against Neu5Gc-glycans with no recognition of any of the NeuSAc-glycans **(****Fig. 8B****),** suggesting that the minor reactivity observed by serum analysis is likely related to cross reactive antibodies. Detailed glycan microarray repertoire analysis also demonstrated a clear change in diversity of anti-Neu5Gc IgG, with preference towards reactivity against Neu5Gc-glycans with α3-linkage in Q4 **(****Fig. 8C****).**

To test whether the increased levels and reactivities of anti-Neu5Gc IgGs were also associated with enhanced binding strength, the affinity equilibrium constant K_{D} was evaluated. Rather than kinetic measurements, and in light of the limited sample obtained by affinity-purification, K_{D} was evaluated through glycan microarray analysis, against a constant concentration of each of the printed Neu5Gc-glycans (at 100 µM), by fitting a plot of response at equilibrium against a wide range of concentrations of the purified antibodies. This analysis revealed no significant differences in the affinities of the antibodies purified from Q4 compared to Q1, despite the much higher yields, although a slight and non-significant decrease in K_{D} was noticed (Fig. 8D). Similar results were obtained from affinity-purified antibodies of women aged 45-60 with quartiles divided according to dairy cow, revealing ~1.3 fold higher antibody yield in Q4 compared to Q1. Analysis of the affinity-purified antibodies on glycan microarray demonstrated high specificity against Neu5Gc-glycans, with altered anti-Neu5Gc IgG repertoire, but no change in affinities of these antibodies. Interestingly, in these affinity purified antibodies profiles, the ratio of total anti-Neu5Gc IgG reactivity against Neu5Gc-glycans with α3-linkages to total anti-Neu5Gc IgG reactivity against Neu5Gc-glycans with α6-linkages were 1.34 in Q1 and 2.04 in Q4 in men aged 45-60, and 1.98 in Q1 and 3.03 in Q4 in women aged 45-60.

### Example 9. Ratio score of anti-Neu5Gc IgG reactivity against glycans with α3-linkage versus α6-linkage

The ratios of the total anti-Neu5Gc IgG reactivity against Neu5Gc-glycans with α3-linkages over total reactivity against Neu5Gc-glycans with α6-linkages (anti-Neu5Gc α3:α6 ratio) were calculated on glycan microarray for the group of Men 45-60 stratified according to their Neu5Gc-intake from red meat. In these men, the ratio differentiate between those who consumed low red meat (Q1) with those of higher Neu5Gc-intake from red meat (Q2-Q4) **(****Fig. 9A****).** The mean anti-Neu5Gc α3:α6 ratio in Men 45-60 who consumed low red meat (Q1) was 0.61±0.28 and in men who consumed higher Neu5Gc-intake from red meat (Q2-Q4) 1.47±0.37. Moreover, the receiver operating characteristic (ROC) curve **(****Fig. 9B****)** showing the diagnostic ability of a method, demonstrates that the results may be used for predicting the presence of high amount of antibodies that are correlated to high risk of developing Neu5Gc related disease or disorder. As can be calculated from **Fig. 9B****,** the AUC was 0.687 ± 0.098 (95% CI 0.494 to 0.879, P=0.0803). Similar results were obtained in comparison between Q1 to Q4 (P=0.0821, AUC 0.73 ± 0.1162, 95% CI 0.5022 to 0.9578).

Altogether, these data provide for the first time a strong association between dietary Neu5Gc and the levels and diversity of anti-Neu5Gc antibodies in humans. Both serum as well as affinity-purified antibodies showed much higher levels of anti-Neu5Gc antibodies with higher Neu5Gc consumption levels, irrespectively of the contributing food source, as well as a change in diversity, but no change in affinity.

### Example 10. Personal dietary recommendations based on Gcemic index

To translate these findings into practical personalized dietary recommendations, we calculated the gram of each food item to consume to reach the ranges of average Neu5Gc consumed in the highest and lowest quartiles for both genders **(Table 6).** The Q1 range is 6937-9443 nmol/day (Min-Max is Q1-Women-Q1-Men), and Q4 range is 19627-25265 nmol/day (Min-Max is Q4-Women-Q4-Men). In addition, we developed a *`Gcemic index"* that is the Neu5Gc content (nmol/gr) in each food item relative to the amount measured in beef (163 nmol/gr). A Gcemic index of 1 means that daily consumption of 58 gr of beef at most is the maximal Q1-Neu5Gc (9443 nmol/day), while daily consumption of at least 120 gr is the minimum Q4-Neu5Gc (19627 nmol/day; **Table 6**). Thus, one can consume 1-2 medium sized beef steaks (225 gr raw meat) per week to fall in Q1 range, while 4-5 steaks weekly is already at Q4 range. A lower Gcemic index (or high inverse Gcemic index; Table 6) means that higher amount of food are needed to be consumed in order to reach the Q1/Q4 ranges. In the Table, Q1 range (Min-Max) refer to amount of food in gram needed to reach Q1 content of Neu5Gc as described above.

**Table 6. Gcemic index**

| **Source** | | **Food item** | **Neu5Gc Average ± sem (nmol/gr)** | **Q1 Range Min - Max (gr)** | **Q4 Range Min - Max (gr)** | **Gcemic index** | **Inverse Gcemic index** |
|---|---|---|---|---|---|---|---|
| Dairy | Buffalo | Mozzarella | 6± 1 | 1223 - 1664 | 3459 - 4453 | 0.03 | 28.90 |
| Meat | Rabbit | Rabbit | 7 ± 4 | 962 - 1310 | 2723 - 3505 | 0.04 | 22.75 |
| Dairy | Cow | Crème fraiche | 11 ± 4 | 623 - 848 | 1762 - 2268 | 0.07 | 14.72 |
| Dairy | Cow | Parmesan | 11 ± 2 | 604 - 822 | 1708 - 2198 | 0.07 | 14.27 |
| Meat | Pig | Pork | 18 ± 3 | 393 - 536 | 1113 - 1433 | 0.11 | 9.30 |
| Dairy | Cow | Plain yogurt | 18 ± 7 | 382 - 519 | 1079 - 1390 | 0.11 | 9.02 |
| Dairy | Cow | Petit Suisse | 18 ± 9 | 378 - 514 | 1069 - 1375 | 0.11 | 8.93 |
| Dairy | Cow | Gouda | 18 ± 6 | 377 - 514 | 1068 - 1375 | 0.11 | 8.92 |
| Dairy | Cow | Milk | 21 ± 4 | 332 - 452 | 939 - 1209 | 0.13 | 7.84 |
| Dairy | Cow | Cheese strainer | 21 ± 8 | 325 - 443 | 920 - 1184 | 0.13 | 7.68 |
| Meat | Cow | Dried sausage | 22 ± 9 | 318 - 433 | 900 - 1159 | 0.13 | 7.52 |
| Dairy | Cow | Fromage blanc | 23 ± 11 | 300 - 409 | 850 - 1094 | 0.14 | 7.10 |
| Dairy | Cow | Cheese spread | 23 ± 13 | 300 - 408 | 848 - 1092 | 0.14 | 7.08 |
| Meat | Pig | Bacon strips | 31 ± 15 | 224 - 304 | 633 - 814 | 0.19 | 5.28 |
| Meat | Cow | Sausages | 32 ± 3 | 219 - 298 | 619 - 796 | 0.19 | 5.17 |
| Dairy | Cow | Camembert | 59 ± 7 | 118 - 161 | 335 - 431 | 0.36 | 2.80 |
| Meat | Lamb | Lamb | 67 ± 58 | 104 - 141 | 294 - 378 | 0.41 | 2.46 |
| Meat | Pig | Pork rillettes | 68 ± 41 | 102- 139 | 289 - 372 | 0.41 | 2.41 |
| Meat | Pig | Ham | 71 ± 48 | 98 - 133 | 276 - 355 | 0.43 | 2.31 |
| Dairy | Cow | Powdered milk | 107 ± 36 | 65 - 88 | 183 - 236 | 0.65 | 1.53 |
| Meat | Cow | Innards | 110 ± 65 | 63 - 86 | 178 - 229 | 0.67 | 1.49 |
| Meat | Cow | Beef tongue | 131 ± 124 | 53 - 72 | 150 - 193 | 0.80 | 1.25 |
| Meat | Pig | Cured ham | 140 ± 89 | 50 - 67 | 140 - 181 | 0.85 | 1.17 |
| **Meat** | **Cow** | **Beef** | **163 ± 17** | **23 - 58** | **120 - 154** | **1.00** | **1.00** |
| Meat | Pig | Sausage | 207 ± 174 | 34 - 46 | 95 - 122 | 1.26 | 0.79 |
| Dairy | Sheep | Yogurt | 277 ± 85 | 25 - 34 | 71 - 91 | 1.69 | 0.59 |
| Dairy | Goat | Feta | 280 ± 15 | 25 - 34 | 70 - 90 | 1.71 | 0.59 |
| Meat | Cow | Pate | 307 ±240 | 23 - 31 | 64 - 82 | 1.87 | 0.53 |
| Dairy | Goat | Soft goat cheese | 336 ± 62 | 21 - 28 | 58 - 75 | 2.05 | 0.49 |
| Dairy | Goat | Roll of cheese | 344 ± 104 | 20 - 27 | 57 - 73 | 2.10 | 0.48 |
| Meat | Pig | Liver | 409 ± 63 | 17 - 23 | 48 - 62 | 2.50 | 0.40 |
| Dairy | Goat | Dry cheese | 533 ± 211 | 13 - 18 | 37 - 47 | 3.25 | 0.31 |
| Dairy | Sheep | Roquefort | 550 ± 13 | 13 - 17 | 36 - 46 | 3.35 | 0.30 |
| Dairy | Sheep | Etorki | 633 ± 78 | 11 - 15 | 31 - 40 | 3.86 | 0.26 |

For example, Mozzarella Gcemic index is 0.03 hence almost 30 times more grams can be consumed compared to beef, while Roquefort Gcemic index is 3.86 suggesting consuming only ¼ the amount of beef can reach Q1/Q4 range. In general, cow dairy has the lowest Gcemic index, while sheep/goat dairy has the highest, while variable in meat.
To evaluate the effect of red meat intake on CRC in different world nations, national per capita meat intake from The Food and Agriculture Organization (FAO) of the United Nations, FAOSTAT database (FAOSTAT. Food and Agriculture Organization of the United Nations (FAO). FAO Statistical Databases Food supply data Available from: http://wwwfaoorg/faostat/en/#data/CL 2020) and CRC age-standardized incidence and mortality rates from GLOBOCAN database (Ferlay J, Ervik M, Lam F, Colombet M, Mery L, Piñeros M, Znaor A, Soerjomataram I, Bray F. Global cancer observatory: cancer today. Lyon, France: International Agency for Research on Cancer Available from: https://gcoiarcfr/today, 2018) were analyzed. We divided countries to quartile according meat consumption and analyzed probability incidence of colorectal cancer and mortality as a result of the cancer. We found that found that gender had no effect on CRC rates in nations of the lowest meat intake quartile (20.25 ± 1.17 gr/capita/day, mean ± sem), but in nations of the highest meat intake quartile (156.4 ± 3.40 gr/capita/day) there were higher incidence and mortality rates in men compared to women **(****Fig. 10****).**

Arbitrarily dividing international meat-cancer risk according to national intake of above/below 120 gr meat per day (lowest beef amount in Q4) shows an increase of 3-fold in incidence and 2.5-fold in mortality in nations that consume >120 gr meat per **(****Fig. 11****).** Interestingly, countries of high beef-meat intake fall among the top 15 CRC incidence and mortality rates, including United States, Australia and France, as well as many counties in South America as Argentina, Brazil, Uruguay and Chile.

The project leading to this application has received funding from the European Research Council (ERC) under the European Union's Horizon 2020 research and innovation program (grant agreement No. 716220).

Although the present invention has been described herein above by way of preferred embodiments thereof, the scope of what protection is sought for, shall be defined by the appended claims.

## Claims

1. A method for determining an antibody repertoire score in a biological sample obtained from a subject comprising:
(i) evaluating the repertoire of the anti-Neu5Gc antibodies in the biological sample obtained from the subject; and
(ii) calculating a ratio of antibodies against Neu5Gcα2-3-linked glycans to antibodies against Neu5Gcα2-6-linked glycans,
wherein the calculated ratio is the antibody repertoire score and wherein the antibody repertoire score value above a control value is indicative to increased likelihood of developing a Neu5Gc related disease or disorder, preferably wherein evaluating the repertoire of the anti-Neu5Gc antibodies in the biological sample comprises determining the amount of antibodies against a set of Neu5Gcα2-3-linked glycans and the amount of antibodies against a set of Neu5Gcα2-6-linked glycans in said biological sample.

2. A method for identifying a subject at increased risk of developing a Neu5Gc related disease or disorder comprising:
(i) calculating the antibody repertoire score according to the method of claim 1; and
(ii) comparing the obtained score to a control antibody repertoire score,
wherein the value of said antibody repertoire score above the value of the control antibody repertoire score is predictive to increased risk of developing the disease or disorder.

3. The method according to claim 1 or 2, wherein the value of the control antibody repertoire score is 0.9.

4. The method of any one of claims 1 to 3, further comprising informing the subject having an increased risk of developing the disease or disorder and providing dietary guidelines designed to reduce the risk.

5. The method of claim 4, wherein the dietary guidelines are **characterized by** at least one of (i) the guidelines comprises guidance to consume no more than 10 µmol/day of Neu5Gc and (ii) the dietary guidelines are based on Neu5Gc content in food as disclosed in Table 6.

6. The method according to any one of claims 1 to 5, wherein (i) measuring the level of anti-Neu5Gc antibodies is performed by an immunoassay, (ii) the biological sample is blood or serum, or (iii) both (i) and (ii).

7. The method according to any one of claims 1 to 6, wherein the Neu5Gc related disease or disorder is selected from cancer, atherosclerotic cardiac disease, and chronic inflammatory conditions.

8. The method according to claim 7, wherein the disease is cancer, preferably wherein the cancer is selected from colorectal cancer and breast cancer.

9. A diagnostic kit for evaluating the repertoire of the anti-Neu5Gc antibodies in a biological sample comprising:
a set of Neu5Gcα2-3-linked glycans;
a set of Neu5Gcα2-6-linked glycans; and
means for quantifying the amount of antibodies bound to Neu5Gcα2-3-linked glycans and to Neu5Gcα2-6-linked glycans.

10. The kit according to claim 9, wherein the kit is **characterized by** at least of:
(i) the set of Neu5Gcα2-3-linked glycans comprises at least 3 glycans selected from glycans having ID number 2, 8, 10, 12, 14, 16, 22, 26, 30, 34, 36, 40, 56, 58, 61, 63 as set forth in Table 1;
(ii) the set of Neu5Gcα2-6-linked glycans comprises at least 3 of the glycans selected from glycans having ID number 4, 6, 18, 20, 24, 28, 32, 38 as set forth in Table 1; and
(iii) the set of Neu5Gcα2-3-linked glycans comprises at least 5 glycans selected from glycans having ID number 2, 8, 10, 12, 14, 16, 22, 26, 30, 34, 36, 40, 56, 58, 61, 63 as set forth in Table 1 and wherein the set of Neu5 Gcα2-6-linked glycans comprises at least 5 of the glycans selected from glycans having ID number 4, 6, 18, 20, 24, 28, 32, 38 as set forth in Table 1.

11. The kit according to any one of claims 9 to 10, wherein the kit is **characterized by** at least one of:
(i) the set of Neu5Gcα2-3-linked glycans and the set of Neu5Gcα2-6-linked glycans are marked by one or more markers, preferably wherein the markers (i) allow quantifying separately antibodies bound to each set; (ii) are configured to be bound to a surface; or (iii) both (i) and (ii);
(ii) the two sets of Neu5Gc glycans are located in one compartment or in two different compartments;
(iii) the means for quantifying the amount of antibodies bound to Neu5Gcα2-3-linked glycans and to Neu5Gcα2-6-linked glycans is an immunoassay;
(iv) (iv) the kit comprises means for determining the ratio between antibodies bound to Neu5Gcα2-3-linked glycans and to Neu5Gcα2-6-linked glycans; and
(v) (v) the two sets of glycans are immobilized on a solid surface or dissolved in a solvent.

12. The kit according to any one of claims 9 to 11, for (i) implementing the method according to claim 1, (ii) for identifying a subject at increased risk of developing a Neu5Gc related disease or disorder, or (iii) for both (i) and (ii).

13. A method for predicting an increased likelihood of developing a Neu5Gc related disease or disorder comprising calculating the antibody repertoire score according to the method of claim 1 and further calculating the intake of NeuSGs based on a collected data on food consumption habits of a subject and combining the results with the antibody repertoire score to calculate an joined likelihood predictive of developing the disease.

14. The method according to claim 13, wherein the Neu5Gc associated disease, disorder or condition is selected from cancer, atherosclerotic cardiac disease, and chronic inflammatory conditions.

## Patentansprüche

1. Verfahren zur Bestimmung eines Antikörper-Repertoire-Scores in einer biologischen Probe, die von einer Person erhalten wurde, wobei das Verfahren umfasst:
(i) Auswerten des Repertoires der Anti-Neu5Gc-Antikörper in der von der Person erhaltenen biologischen Probe; und
(ii) Berechnen eines Verhältnisses von Antikörpern gegen Neu5Gcα2-3-verknüpfte Glykane zu Antikörpern gegen Neu5Gcα2-6-verknüpfte Glykane,
wobei das berechnete Verhältnis der Antikörper-Repertoire-Score ist und wobei der Antikörper-Repertoire-Score-Wert oberhalb eines Kontrollwertes auf eine erhöhte Wahrscheinlichkeit der Entwicklung einer Neu5Gc-verwandten Krankheit oder Störung hinweist, vorzugsweise wobei die Auswertung des Repertoires der Anti-Neu5Gc-Antikörper in der biologischen Probe die Bestimmung der Menge an Antikörpern gegen einen Satz von Neu5Gcα2-3-verknüpften Glykanen und der Menge an Antikörpern gegen einen Satz von Neu5Gcα2-6-verknüpften Glykanen in der biologischen Probe umfasst.

2. Verfahren zur Identifizierung einer Person mit erhöhtem Risiko, eine Neu5Gc-bezogene Krankheit oder Störung zu entwickeln, wobei das Verfahren umfasst:
(i) Berechnen des Antikörper-Repertoire-Scores gemäß dem Verfahren nach Anspruch 1; und
(ii) Vergleichen des erhaltenen Wertes mit einem Kontroll-Antikörper-Repertoire-Score,
wobei der Wert des Antikörper-Repertoire-Scores, der über dem Wert des Kontroll-Antikörper-Repertoire-Scores liegt, ein erhöhtes Risiko vorhersagt, die Krankheit oder Störung zu entwickeln.

3. Verfahren nach Anspruch 1 oder 2, worin der Wert des Kontroll-Antikörper-Repertoire-Scores 0,9 beträgt.

4. Verfahren nach einem der Ansprüche 1 bis 3, welches ferner das Informieren der Person, die ein erhöhtes Risiko hat, die Krankheit oder Störung zu entwickeln, und das Bereitstellen von Ernährungsrichtlinien umfasst, die darauf ausgelegt sind, das Risiko zu verringern.

5. Verfahren nach Anspruch 4, wobei die Ernährungsrichtlinien **dadurch gekennzeichnet sind, dass** (i) die Richtlinien die Anweisung enthalten, nicht mehr als 10 µmol/Tag an Neu5Gc zu konsumieren, und (ii) die Ernährungsrichtlinien auf dem Neu5Gc-Gehalt in Lebensmitteln basieren, wie in Tabelle 6 offenbart.

6. Verfahren nach einem der Ansprüche 1 bis 5, wobei (i) die Messung des Gehalts an Anti-NeuSGc-Antikörpern durch einen Immunoassay durchgeführt wird, (ii) worin die biologische Probe Blut oder Serum ist oder (iii) sowohl (i) als auch (ii) ist.

7. Verfahren nach einem der Ansprüche 1 bis 6, worin die mit Neu5Gc zusammenhängende Krankheit oder Störung ausgewählt ist aus Krebs, atherosklerotischer Herzerkrankung und chronischen Entzündungszuständen.

8. Verfahren nach Anspruch 7, worin es sich bei der Krankheit um Krebs handelt und der Krebs vorzugsweise ausgewählt ist unter kolorektalem Krebs und Brustkrebs.

9. Diagnostik-Kit zur Bewertung des Repertoires der Anti-Neu5Gc-Antikörper in einer biologischen Probe, welches umfasst:
einen Satz von Neu5Gcα2-3-verknüpften Glykanen;
einen Satz von Neu5Gcα2-6-verknüpften Glykanen; und
Mittel zur Quantifizierung der Menge von Antikörpern, die an Neu5Gcα2-3-verknüpfte Glykane und an Neu5Gcα2-6-verknüpfte Glykane gebunden sind.

10. Kit nach Anspruch 9, worin das Kit mindestens durch eines gekennzeichnet ist, dass:
(i) der Satz von Neu5Gcα2-3-verknüpften Glykanen mindestens 3 Glykane umfasst, ausgewählt unter Glykanen mit der ID-Nummer 2, 8, 10, 12, 14, 16, 22, 26, 30, 34, 36, 40, 56, 58, 61, 63, wie in Tabelle 1 angegeben;
(ii) der Satz von Neu5Gcα2-6-verknüpften Glykanen mindestens 3 der Glykane umfasst, ausgewählt unter Glykanen mit den ID-Nummern 4, 6, 18, 20, 24, 28, 32, 38, wie in Tabelle 1 dargelegt; und
(iii) der Satz von Neu5Gcα2-3-verknüpften Glykanen mindestens 5 Glykane umfasst, ausgewählt unter Glykanen mit der ID-Nummer 2, 8, 10, 12, 14, 16, 22, 26, 30, 34, 36, 40, 56, 58, 61, 63, wie in Tabelle 1 dargelegt, und wobei der Satz von Neu5Gcα2-6-verknüpften Glykanen mindestens 5 der Glykane umfasst, ausgewählt unter Glykanen mit der ID-Nummer 4, 6, 18, 20, 24, 28, 32, 38, wie in Tabelle 1 dargelegt.

11. Kit nach einem der Ansprüche 9 bis 10, worin das Kit mindestens durch eines gekennzeichnet ist, dass:
(i) der Satz von Neu5Gcα2-3-verknüpften Glykanen und der Satz von Neu5Gcα2-6-verknüpften Glykanen durch einen oder mehrere Marker markiert sind, vorzugsweise worin die Marker (i) eine getrennte Quantifizierung von Antikörpern, die an jeden Satz gebunden sind, ermöglichen; (ii) so konfiguriert sind, dass sie an eine Oberfläche gebunden werden; oder (iii) sowohl (i) als auch (ii);
(ii) die beiden Sätze von NeuSGc-Glykanen sich in einem Kompartiment oder in zwei verschiedenen Kompartimenten befinden;
(iii) das Mittel zur Quantifizierung der Menge an Antikörpern, die an Neu5Gcα2-3-verknüpfte Glykane und an Neu5Gcα2-6-verknüpfte Glykane gebunden sind, ein Immunoassay ist;
(iv) das Kit Mittel zur Bestimmung des Verhältnisses zwischen Antikörpern umfasst, die an Neu5Gcα2-3-verknüpfte Glykane und an Neu5Gcα2-6-verknüpfte Glykane gebunden sind; und
(v) die beiden Sätze von Glykanen auf einer festen Oberfläche immobilisiert oder in einem Lösungsmittel gelöst sind.

12. Kit nach einem der Ansprüche 9 bis 11 (i) zur Durchführung des Verfahrens nach Anspruch 1, (ii) zur Identifizierung einer Person mit erhöhtem Risiko für die Entwicklung einer Neu5Gc-bezogenen Krankheit oder Störung, oder (iii) sowohl für (i) als auch für (ii).

13. Verfahren zur Vorhersage einer erhöhten Wahrscheinlichkeit, eine mit Neu5Gc zusammenhängende Krankheit oder Störung zu entwickeln, wobei das Verfahren die Berechnung des Antikörper-Repertoire-Scores gemäß dem Verfahren nach Anspruch 1 und ferner die Berechnung der Aufnahme von NeuSGs auf der Grundlage von gesammelten Daten über die Ernährungsgewohnheiten einer Person und die Kombination der Ergebnisse mit dem Antikörper-Repertoire-Score zur Berechnung einer gemeinsamen Wahrscheinlichkeit umfasst, die die Entwicklung der Krankheit vorhersagt.

14. Verfahren nach Anspruch 13, worin die Neu5Gc-assoziierte Krankheit, Störung oder der Zustand ausgewählt ist unter Krebs, atherosklerotischer Herzerkrankung und chronischen Entzündungszuständen.

## Revendications

1. Méthode de détermination d'un score de répertoire d'anticorps dans un échantillon biologique obtenu à partir d'un sujet, comprenant:
(i) évaluer le répertoire des anticorps anti-Neu5Gc dans l'échantillon biologique obtenu à partir du sujet; et
(ii) calculer un rapport entre les anticorps dirigés contre les glycanes liés à Neu5Gcα2-3 et les anticorps dirigés contre les glycanes liés à Neu5Gcα2-6,
dans lequel le rapport calculé est le score de répertoire d'anticorps et dans lequel la valeur du score de répertoire d'anticorps supérieure à une valeur de contrôle indique une probabilité accrue de développer une maladie ou un trouble lié au Neu5Gc, de préférence dans lequel l'évaluation du répertoire des anticorps anti-Neu5Gc dans l'échantillon biologique comprend la détermination de la quantité d'anticorps contre un ensemble de glycanes liés à Neu5Gcα2-3 et la quantité d'anticorps contre un ensemble de glycanes liés à Neu5Gcα2-6 dans ledit échantillon biologique.

2. Méthode d'identification d'un sujet présentant un risque accru de développer une maladie ou un trouble lié au Neu5Gc, comprenant:
(i) calculer le score de répertoire d'anticorps selon la méthode de la revendication 1; et
(ii) comparer le score obtenu à un score de répertoire d'anticorps de contrôle,
dans lequel la valeur de ce score de répertoire d'anticorps supérieure à la valeur du score de répertoire d'anticorps de contrôle est prédictive d'un risque accru de développer la maladie ou le trouble.

3. Méthode selon la revendication 1 ou 2, dans laquelle la valeur du score du répertoire d'anticorps de contrôle est de 0,9.

4. Méthode selon l'une des revendications 1 à 3, comprenant en outre informer le sujet présentant un risque accru de développer la maladie ou le trouble et fournir des recommandations alimentaires destinées à réduire le risque.

5. Méthode selon la revendication 4, dans laquelle les recommandations alimentaires sont **caractérisées par** au moins l'une des deux choses suivantes: (i) les recommandations comprennent l'indication de ne pas consommer plus de 10 µmol/jour de Neu5Gc et (ii) les recommandations alimentaires sont basées sur la teneur en Neu5Gc dans les aliments, comme indiqué dans le tableau 6.

6. Méthode selon l'une des revendications 1 à 5, dans laquelle (i) la mesure du niveau d'anticorps anti-Neu5Gc est effectuée par un test immunologique, (ii) l'échantillon biologique est du sang ou du sérum, ou (iii) à la fois (i) et (ii).

7. Méthode selon l'une des revendications 1 à 6, dans laquelle la maladie ou le trouble lié à Neu5Gc est choisi parmi le cancer, les maladies cardiaques athérosclérotiques et les conditions inflammatoires chroniques.

8. Méthode selon la revendication 7, dans laquelle la maladie est un cancer, de préférence un cancer colorectal ou un cancer du sein.

9. Kit diagnostique pour évaluer le répertoire des anticorps anti-Neu5Gc dans un échantillon biologique comprenant:
un ensemble de glycanes liés au Neu5Gcα2-3;
un ensemble de glycanes liés au Neu5Gcα2-6; et
des moyens pour quantifier la quantité d'anticorps liés aux glycanes liés au Neu5Gcα2-3 et aux glycanes liés au Neu5Gcα2-6.

10. Kit selon la revendication 9, dans lequel le kit est **caractérisé par** au moins:
(i) l'ensemble de glycanes liés à Neu5Gcα2-3 comprend au moins 3 glycanes choisis parmi les glycanes ayant les numéros d'identification 2, 8, 10, 12, 14, 16, 22, 26, 30, 34, 36, 40, 56, 58, 61, 63 comme indiqué dans le tableau 1;
(ii) l'ensemble de glycanes liés à Neu5Gcα2-6 comprend au moins 3 des glycanes choisis parmi les glycanes ayant les numéros d'identification 4, 6, 18, 20, 24, 28, 32, 38 comme indiqués dans le tableau 1; et
(iii) l'ensemble de glycanes liés à Neu5Gcα2-3 comprend au moins 5 glycanes choisis parmi les glycanes ayant les numéros d'identification 2, 8, 10, 12, 14, 16, 22, 26, 30, 34, 36, 40, 56, 58, 61, 63 comme indiqués dans le tableau 1 et dans lequel l'ensemble de glycanes liés à Neu5Gcα2-6 comprend au moins 5 des glycanes choisis parmi les glycanes ayant les numéros d'identification 4, 6, 18, 20, 24, 28, 32, 38 comme indiqués dans le tableau 1.

11. Kit selon l'une des revendications 9 à 10, dans lequel le kit est **caractérisé par** au moins l'un des éléments suivants:
(i) l'ensemble de glycanes liés à Neu5Gcα2-3 et l'ensemble de glycanes liés à Neu5Gcα2-6 sont marqués par un ou plusieurs marqueurs, de préférence dans lesquels les marqueurs (i) permettent de quantifier séparément les anticorps liés à chaque ensemble; (ii) sont configurés pour être liés à une surface; ou (iii) à la fois (i) et (ii);
(ii) les deux ensembles de glycanes Neu5Gc sont situés dans un compartiment ou dans deux compartiments différents;
(iii) le moyen de quantifier la quantité d'anticorps liés aux glycanes liés à Neu5Gcα2-3 et aux glycanes liés à Neu5Gcα2-6 est un essai immunologique;
(iv) le kit comprend un moyen de déterminer le rapport entre les anticorps liés aux glycanes liés à Neu5Gcα2-3 et aux glycanes liés à Neu5Gcα2-6; et
(v) les deux ensembles de glycanes sont immobilisés sur une surface solide ou dissous dans un solvant.

12. Kit selon l'une des revendications 9 à 11, pour (i) mettre en oeuvre la méthode selon la revendication 1, (ii) identifier un sujet présentant un risque accru de développer une maladie ou un trouble lié à Neu5Gc, ou (iii) pour (i) et (ii) à la fois.

13. Méthode de prédiction d'une probabilité accrue de développer une maladie ou un trouble lié aux Neu5Gc, comprenant calculer le score de répertoire d'anticorps selon la méthode de la revendication 1, calculer la consommation de Neu5G sur la base des données collectées sur les habitudes de consommation alimentaire d'un sujet et combiner des résultats avec le score de répertoire d'anticorps pour calculer une probabilité jointe de prédire le développement de la maladie.

14. Méthode selon la revendication 13, dans laquelle la maladie, le trouble ou l'état associé aux Neu5Gc est choisi parmi le cancer, les maladies cardiaques athérosclérotiques et les états inflammatoires chroniques.
